# EUROPEAN PATENT SPECIFICATION

(11) **EP 3 673 045 B1**
(45) Date of publication and mention of the grant of the patent: **17.01.2024**
(21) Application number: 18786718.9
(22) Date of filing: 10.10.2018
(51) Int. Cl.: C12N 5/00, C12P 21/00

(54) **PERFUSION MEDIUM**
PERFUSIONSMEDIUM
MILIEU DE PERFUSION

(30) Priority: 13.10.2017 US 201762571915 P
(43) Date of publication of application: 01.07.2020
(73) Proprietor: Boehringer Ingelheim International GmbH, 55216 Ingelheim am Rhein (DE)
(72) Inventor: LIN, Henry, Ridgefield CT 06877-0368 (US); WANG, Samantha, Ridgefield CT 06877-0368 (US); ZHENG, Lili, Fremont, CA 94555 (US)
(74) Representative: Oetke, Cornelia
(86) International application number: PCT/EP2018/077555
(87) International publication number: WO 2019/072889

(56) References cited:
- EP-A1- 1 160 315
- WO-A1-2011/067465
- WO-A1-2014/109858
- WO-A1-2015/095651
- ADAM ELHOFY: "Novel Cell-Ess supplement used as a feed or as an initial boost to CHO serum free media results in a significant increase in protein yield and production", COMPUTATIONAL AND STRUCTURAL BIOTECHNOLOGY JOURNAL, vol. 14, 1 January 2016 (2016-01-01), pages 319-324, XP055532350, Sweden ISSN: 2001-0370, DOI: 10.1016/j.csbj.2016.07.001
- SHEN C F ET AL: "Micro-quantitation of lipids in serum-free cell culture media: a critical aspect is the minimization of interference from medium components and chemical reagents", JOURNAL OF CHROMATOGRAPHY B: BIOMEDICAL SCIENCES & APPLICATI, ELSEVIER, AMSTERDAM, NL, vol. 810, no. 1, 15 October 2004 (2004-10-15), pages 119-127, XP004556483, ISSN: 1570-0232, DOI: 10.1016/J.JCHROMB.2004.07.020
- RAJALA KRISTIINA ET AL: "Effects of the physiochemical culture environment on the stemness and pluripotency of human embryonic stem cells", STEM CELL STUDIES,, vol. 1, no. 1, 1 January 2011 (2011-01-01) , pages 17-27, XP009509867, ISSN: 2038-9566, DOI: 10.4081/SCS.2011.E3
- ALISON COLQUHOUN ET AL: "Regulation of tumour cell fatty acid oxidation by n-6 polyunsaturated fatty acids", IUBMB LIFE, vol. 44, no. 1, 1 January 1998 (1998-01-01), pages 143-150, XP55534522, ISSN: 1521-6543, DOI: 10.1080/15216549800201152
- SARAH WULHFARD ET AL: "Valproic acid enhances recombinant mRNA and protein levels in transiently transfected Chinese hamster ovary cells", JOURNAL OF BIOTECHNOLOGY, vol. 148, no. 2-3, 1 July 2010 (2010-07-01), pages 128-132, XP55532641, AMSTERDAM, NL ISSN: 0168-1656, DOI: 10.1016/j.jbiotec.2010.05.003
- WILLIAM C. YANG ET AL: "Addition of Valproic Acid to CHO Cell Fed-Batch Cultures Improves Monoclonal Antibody Titers", MOLECULAR BIOTECHNOLOGY, vol. 56, no. 5, 1 May 2014 (2014-05-01), pages 421-428, XP55532643, ISSN: 1073-6085, DOI: 10.1007/s12033-013-9725-x
- CHI-HSIEN LIU ET AL: "Pentanoic acid, a novel protein synthesis stimulant for chinese hamster ovary (CHO) cells", JOURNAL OF BIOSCIENCE AND BIOENGINEERING, vol. 91, no. 1, 1 January 2001 (2001-01-01), pages 71-75, XP055532644, NL ISSN: 1389-1723, DOI: 10.1016/S1389-1723(01)80114-6
- CORONEL JULIANA ET AL: "Valeric acid supplementation combined to mild hypothermia increases productivity in CHO cell cultivations", BIOCHEMICAL ENGINEERING JOURNAL, ELSEVIER, AMSTERDAM, NL, vol. 114, 29 June 2016 (2016-06-29), pages 101-109, XP029735260, ISSN: 1369-703X, DOI: 10.1016/J.BEJ.2016.06.031
- JIN HYOUNG PARK ET AL: "Valeric acid induces cell cycle arrest at G1 phase in CHO cell cultures and improves recombinant antibody productivity", BIOTECHNOLOGY JOURNAL, vol. 11, no. 4, 11 December 2015 (2015-12-11), pages 487-496, XP55341970, DE ISSN: 1860-6768, DOI: 10.1002/biot.201500327
- CHEN Z-L ET AL: "Temperature shift as a process optimization step for the production of pro-urokinase by a recombinant Chinese hamster ovary cell line in high-density perfusion culture", JOURNAL OF BIOSCIENCE AND BIOENGINEE, ELSEVIER, AMSTERDAM, NL, vol. 97, no. 4, 1 January 2004 (2004-01-01), pages 239-243, XP008105293, ISSN: 1389-1723, DOI: 10.1016/S1389-1723(04)70198-X
- ZHIMEI DU ET AL: "Use of a small molecule cell cycle inhibitor to control cell growth and improve specific productivity and product quality of recombinant proteins in CHO cell cultures : Simultaneously Control Cell Growth and Improve Specific Productivity and Product Quality", BIOTECHNOLOGY AND BIOENGINEERING, vol. 112, no. 1, 1 January 2015 (2015-01-01), pages 141-155, XP055532325, US ISSN: 0006-3592, DOI: 10.1002/bit.25332
- J.C. SEEGERS ET AL: "Effects of gamma-linolenic acid and arachidonic acid on cell cycle progression and apoptosis induction in normal and transformed cells", PROSTAGLANDINS LEUKOTRIENES AND ESSENTIAL FATTY ACIDS., vol. 56, no. 4, 1 April 1997 (1997-04-01), pages 271-280, XP055532697, EDINBURGH ISSN: 0952-3278, DOI: 10.1016/S0952-3278(97)90570-6
- KUMUDIKA I DE SILVA ET AL: "Prostaglandin E2 mediates growth arrest in NFS-60 cells by down-regulating interleukin-6 receptor expression", THE BIOCHEMICAL JOURNAL, , vol. 370, no. Pt 1 15 February 2003 (2003-02-15), pages 315-321, XP009509964, DOI: 10.1042/BJ20021512 Retrieved from the Internet: URL:http://www.biochemj.org/content/ppbioc hemj/370/1/315.full.pdf

## Description

### TECHNICAL FIELD

The invention relates to improved perfusion cell culture media and methods that achieve greater cell specific productivity and better sustained and/or maintained viability relative to state of the art methods. The invention further relates to the use of new and improved cell culture media lipid-based additives that more effectively maintain cell culture viability and increase cell specific productivity through, in part, effectively suppressing cell growth during cell culture, e.g., during production phase of perfusion cell culture. More in particular, the invention relates to the use of effective amounts of one or more lipid or lipid metabolite additives, including linoleic acid, arachidonic acid, and prostaglandin E2, during perfusion cell culture methods (e.g., during production phase) which results in growth suppression with concomitant reduced cell bleed, maintained high cell viability, and/or increased cell specific product titers during production. More in particular, the invention relates to cell culture media comprising effective amounts of one or more of linoleic acid, arachidonic acid, and prostaglandin E2 for use during perfusion cell culture methods, and in particular, during production phase, which result in improved and/or maintained cell viability and/or increased cell specific productivity. The invention also relates to methods for suppressing the growth of cells in the perfusion state by increasing the synthesis of linoleic acid, arachidonic acid, and/or prostaglandin E2 by genetic and/or biochemical means.

### BACKGROUND

Three methods are typically used in commercial processes for the production of recombinant proteins by mammalian cell culture: batch culture, fed-batch culture, and perfusion culture.

Perfusion based methods offer potential improvements over batch and fed-batch methods, including improved product quality and stability, improved scalability, and increased cell specific productivity. Unlike batch and fed-batch bioreactors, perfusion systems involve the continuous removal of spent media. By continuously removing spent media and replacing it with new media, the levels of nutrients are better maintained which simultaneously optimizes growth conditions and removes cell waste products. The diminished waste products reduce toxicity to the cells and the expression products. Thus, perfusion bioreactors typically result in significantly less protein degradation and thus, a higher quality product. Product can also be harvested and purified much more quickly and continuously, which is particularly effective when producing a product that is unstable.

Perfusion bioreactors are also more easily scalable. As compared to traditional batch or fed-batch systems, perfusion bioreactors offer several advantages with regard to scalability and/or increasing demand. For one, perfusion bioreactors are smaller in size and can produce the same productivity (i.e., product yield) with less volume. It is accepted that perfusion bioreactors can function at 5- to 20-fold concentrations compared to fed-batch bioreactors. For example, a 100-liter perfusion bioreactor can produce the same product yield as a 1,000-liter fed-batch bioreactor. Therefore, the use of a 1,000-liter perfusion bioreactor could conceivably replace a typical 10,000-liter traditional fed-batch bioreactor without negatively impacting the overall productivity. This significant advantage translates into smaller space requirements when expanding production. This may also translate into an array of advantages relating to lower operational utilities, less infrastructure, less labor, reduced complexity of equipment, continuous harvesting, and increased product yields.

Achieving high cell culture densities accounts for part of the greater productivity of perfusion systems. In a typical large scale fed-batch commercial cell culture process, cell densities of 10 - 50 x 10⁶ cells/mL can be reached. However, with perfusion-based bioreactors, extreme cell densities of >1 x 10⁸ cells/mL have been achieved. In addition, in perfusion mode, high cell numbers are sustained for much longer periods of time through the continuous replenishment of spent media. The higher cell densities for increased periods of time in perfusion bioreactors accounts in part for their more efficient performance.

Typical perfusion cultures begin with a batch culture start-up lasting for a day or more to enable rapid initial cell growth and biomass accumulation, followed by continuous, step-wise and/or intermittent addition of fresh perfusion media to the culture and simultaneous removal of spent media with retention of cells throughout the growth and production phases of the culture. Various methods, such as sedimentation, centrifugation, or filtration, can be used to remove spent media, while maintaining the cells. Perfusion flow rates of a fraction of a working volume per day up to many multiple working volumes per day have been utilized.

While continuous perfusion systems have numerous advantages over traditional fed-batch and batch systems, many challenges still remain before perfusion bioreactors become more widely accepted and utilized in the biologics manufacturing industry. For example, perfusion bioreactors consume a significantly greater volume of media than traditional fed-batch systems due to the continuous cycle of removal and replenishment of media. In addition, there is a general lack of adequate sensor technology for the real-time direct evaluation of product quality, such as protein folding, aggregation, glycosylation, oxidation, and contamination-which presently can only be determined by sampling and analysis. Having such technology capability would greatly support the operation of long term steady-state perfusion. Another limitation is the general lack of available perfusion bioprocessing modeling software, which is largely due to lack of robust data sources regarding perfusion-based systems. See E. Langer and R. Rader, "Continuous Bioprocessing and Perfusion: Wider Adoption Coming as Bioprocessing Matures," BioProcess J, 2014; 13(1): 43-49.

One vitally important area requiring improvement is to develop better perfusion bioreactor media. Relatively little scientific data is available on the nutritional requirements of cells grown in perfusion as compared to the well-established knowledge of cells grown in batch culture. See B. Sargent, "Are Perfusion Cell Culture Systems the Future for Cell-Culture Based Biomanufacturing," The Cell Culture Dish, February 3, 2012, available online. To some degree, recent improvements in media have achieved higher viable cell densities and, in turn, higher titers in perfusion processes. However, higher viable cell densities can create other problems. In particular, these elevated viable cell densities can become unsustainable resulting in reduced culture viability, shortened perfusion culture runs, and concomitantly, reduced productivity. Thus, cell culture media design is of key importance in achieving greater cell health, viability, and productivity while performing perfusion cell culture. Improvements to perfusion cell culturing media design and culturing methods are needed to overcome the deficiencies and challenges in the art.

Another problem facing continuous perfusion cell culture systems is the challenge of maintaining a constant viable cell density, and by consequence, a healthier and more productive cell culture. This has typically been addressed by allowing for "cell bleed." During cell bleeding, cells are removed and discarded as waste at a rate sufficient to allow for a steady state perfusion cell culture. In turn, this keeps viable cell density constant. However, cell bleeding is wasteful since the removed cells are discarded even though they contain the product of interest. Therefore, any amount of cell bleeding negatively impacts process efficiency, product recovery and most importantly product loss. The cell bleed rate is determined by rate of cell growth. A faster doubling time also necessitates a higher cell bleed to maintain constant cell density, and consequently, more waste.

As an alternative to cell bleeding, others have tried chemical additives to slow the rate of cell growth. For example, Du et al. (Biotechnology and Bioengineering, Vol. 112, No. 1, January 2015) reported the use of a small molecule cell cycle inhibitor to control growth and improve cell culture productivity. A similar disclosure is found in WO 2014/109858, which discloses the use of CDK4 inhibitor in cell culture such as batch, fed-batch and perfusion culture. Du et al. further teaches that CDK4/6 inhibitors specifically inhibit the cell cycle without affecting other cellular targets. Other cell growth inhibitors are not disclosed. Moreover, Chen et al., (Journal of Bioscience and Bioengineering (2004), 97(4): 239-243) examined the potential of temperature shift as a tool in the optimization of the perfusion culture of recombinant CHO cells for the production of pro-urokinase.

Thus, additional methods and agents that are effective to suppress cell growth in the perfusion state and avoid the need for cell bleeding would significantly help advance the art.

Still another problem facing perfusion bioreactors is an overall lack of knowledge regarding nutritional supplements. Batch cell culture media has long involved the use of nutritional supplements or additives to help improve various aspects of cell culture, including cell viability, growth, and productivity. See Gorfien et al., "Optimized Nutrient Additives for Fed-Batch Cultures," BioPharm International, April 2003, pp. 34-40. However, as noted above, media design for continuous perfusion culture systems is far less developed.

With regard to lipid-based supplements, lipid components and liposomes have been described previously for use in cell culture, but with limited success. For example, Hams et al. (Proc Natl Acad Sci USA 78:5588-92, 1981) discloses culturing human fibroblasts in media comprising different lipid components, e.g., a liposome comprising cholesterol, lecithin or purified phosphatidylcholine, sphingomyelin and vitamin E. However, Hams et al. describes that the liposomes are not stable in that they need to be made within 24 hours of use, and shows that simply using the liposomes in complete media does not promote cell growth. Furthermore, the culture system is not perfusion-based.

Lipid-based supplements are also described in Spens et al. (Biotechnol Prog 21:87-95, 2005). Spens et al. describe culture of NS0 myeloma cells in a protein-free media further comprising cholesterol, cyclodextrin, phosphatidylcholine, vitamin E, ferric citrate, pluronic and amino acids, among other things. However, Spens et al. describes that at high levels lipids precipitate out of solution. Furthermore, the culture system is not perfusion-based.

WO 94/23572 A2 ("Cell Culturing Method and Medium") relates to a method for producing a cell culture of liver cells, among other types of animal cells. The reference relates to preparing the cells by mincing a tissue sample and growing cells in a tissue culture in a medium that may include linoleic acid. However, the purpose of the cell culture in this reference is to proliferate cells, not to inhibit cell proliferation. Cell culture is also not a perfusion culture. Thus, this reference does not teach or suggest the use of linoleic acid as a nutritional supplement for use in a perfusion culture, nor does it teach or suggest the use of linoleic acid to suppress cell growth in the perfusion state.

WO 98/04681 A2 ("Chondrocyte Media Formulations and Culture Procedures") relates to growth media and procedures for preparing proliferating a culture of chondrocytes isolated from an animal. The reference discloses various serum-free defined cell culture media that can include linoleic acid. However, the purpose of the cell culture in this reference is to proliferate cells, not to inhibit cell proliferation. Cell culture is also not a perfusion culture. Thus, this reference does not teach or suggest the use of linoleic acid as a nutritional supplement for use in a perfusion culture, nor does it teach or suggest the use of linoleic acid to suppress cell growth in the perfusion state.

WO 2000/027996 ("Serum Free Medium for Chondrocyte-Like Cells") relates to serum free media for growth and proliferation of chondrocytes and mesenchymal stem cells in culture. The reference discloses various serum-free defined cell culture media that can include linoleic acid. However, the purpose of the cell culture in this reference is to proliferate cells, not to inhibit cell proliferation. Cell culture is also not a perfusion culture. Thus, this reference does not teach or suggest the use of linoleic acid as a nutritional supplement for use in a perfusion culture, nor does it teach or suggest the use of linoleic acid to suppress cell growth in the perfusion state.

WO 2003/064598 ("Serum-Free Media for Chondrocytes and Methods of Use") relates to serum free media for growth and proliferation of chondrocytes in culture. The reference discloses various serum-free defined cell culture media that can include linoleic acid and arachidonic acid. However, the purpose of the cell culture in this reference is to propagate cells, not to inhibit cell proliferation. Cell culture is also not a perfusion culture. Thus, this reference does not teach or suggest the use of linoleic acid or arachidonic acid as a nutritional supplement for use in a perfusion culture, nor does it teach or suggest the use of linoleic acid to suppress cell growth in the perfusion state.

Also, Elhofy et al. (Computational and Structural Biotechnology Journal (2016) 14: 319-324), WO 2015/095651 A1, Shen et al. (Journal of Chromatography B: Biomedical Sciences & Applications, (2004) 810(1): 119-127), EP 1 160 315 A1, WO 2011/067465 A1, Rajala et al., (Stem Cell Studies (2011), 1(1): 17-27), Colquhoun et al. (Biochemistry and Molecular Biology International (1998), 44(1): 143-150), Seegers et al., (Prostaglandins, Leukotrienes and Essential Fatty Acids (1997), 56(4): 271-280) and Kumudika et al., (The Biochemical Journal (2003), 370: 315-321) disclose lipid supplements, but not in the context of heterologous protein expression in perfusion cell culture and/or at different concentration ranges. Further, short fatty acids, such as valproic and valeric acid as supplements for batch or fed-batch culture are disclosed in Wulhfard et al., (Journal of Biotechnology 148 (2010): 128-132), Yang et al., (Molecular Biotechnology (2014), 56(5): 421-428), Liu et al., (Journal of Bioscience and Bioengineering (2001), 91(1): 71-75), Coronel et al., (Biochemical Engineering Journal (2016), 114: 101-109) and Park et al., (Biotechnology Journal (2006), 11(4):487-496).

In view of the challenges with perfusion cell culture noted in the art, additional media supplements or additives that are effective in suppressing cell growth in the perfusion state and avoid the need for cell bleeding would significantly help advance the art.

### SUMMARY OF THE INVENTION

The present invention relates in part to the discovery that certain lipids and lipid metabolites when included singularly or in combination in cell culture media, e.g., linoleic acid, arachidonic acid, and prostaglandin E2, are effective at causing an increase in cell specific productivity and/or are effective in maintaining or sustaining high cell viability and/or suppressing cell growth of the cell culture. These agents can be used in connection with any type of cell culture system, including batch, fed-batch, and continuous perfusion cell culture systems. In the context of the present invention, the cell culture system is a continuous perfusion cell culture system.

It was found that the lipids and lipid metabolites described herein, e.g., linoleic acid, arachidonic acid, and prostaglandin E2, were effective in suppressing cell growth, and that this growth suppression led to an increase in cell specific productivity and helped in maintaining high viability in a cell culture.

With regard to perfusion cell cultures, the cell growth suppression by the lipids and/or lipid metabolites described herein (e.g., linoleic acid, arachidonic acid, and prostaglandin E2) not only led to an increase in cell specific productivity and sustained high cell viability in the perfusion cell culture, while reducing or eliminating the need to employ cell bleeding techniques during the perfusion state to otherwise maintain the cells in a steady state of growth. In other words, the suppression of cell growth in the perfusion state by the lipids and lipid metabolites of the invention increases overall cell specific productivity and helps to maintain high cell viability while minimizing or eliminating wasteful and undesirable cell bleeding techniques.

As indicated above, a wasteful cell bleed can be utilized to maintain a sustainable viable cell density and preserve viability in connection with continuous cell culturing processes. In continuous processes, a large proportion of the culture medium and hence product can be lost due to the technique of cell bleeding, which siphons off proliferating cells and medium in order to maintain a constant, sustainable viable cell density within the bioreactor. Up to one-third of harvestable material can be lost due to cell bleeding techniques. Using cell bleeding therefore decreases the product yield per run as the product within the portion removed by cell bleeding is not harvested. In order to decrease the volume of culture removed by cell bleeding and thus retain more supernatant for harvesting it is advantageous to inhibit cell proliferation once the desired viable cell density is reached in the production phase. There is a need for controlling cell growth once an optimal variable cell density has been obtained, without utilizing wasteful cell bleeding techniques, thereby increasing product recovered per perfusion run and generating a more efficient method for operating perfusion processes.

As demonstrated in the specific embodiments of the Examples herein, the present invention provides a perfusion culture medium comprising one or more lipids or lipid metabolites comprising 500-2000 µM linoleic acid, 100-600 µM arachidonic acid, and/or 0.0001-100 µM prostaglandin E2. The invention further provides methods of culturing mammalian cells, e.g., Chinese Hamster Ovary (CHO) cells, using said medium, wherein the cell culturing method is a perfusion cell culture, e.g., a continuous perfusion culturing method. The Examples demonstrate that linoleic acid, arachidonic acid, and prostaglandin E2-alone or in combination-have the same effect on mammalian cells in perfusion cell culture, wherein said effect is growth suppression with a concomitant increase in production of product, without the need for wasteful cell bleeding.

Thus, the following aspects of the disclosure are provided herein.

In a first aspect of the disclosure, a method of culturing mammalian cells expressing a heterologous protein in a perfusion cell culture is provided comprising culturing mammalian cells expressing a heterologous protein in a culture medium comprising an effective amount of one or more lipids or lipid metabolites wherein the one or more lipids or lipid metabolites comprises 500-2000 µM linoleic acid, 100-600 µM arachidonic acid, and/or 0.0001-100 µM prostaglandin E2. In certain embodiments, the lipid or lipid metabolite or combination thereof results in growth suppression and/or increased cell specific productivity. In certain other embodiments relating to perfusion cell culture, the growth suppression and/or increased cell specific productivity may reduce or eliminate the need for cell bleeding to maintain or achieve a steady state.

In a second aspect of the disclosure, a method of culturing mammalian cells expressing a heterologous protein in a perfusion cell culture is provided comprising culturing mammalian cells expressing a heterologous protein in a perfusion culture medium comprising an effective amount of 500-2000 µM linoleic acid.

In a third aspect of the disclosure, a method of culturing mammalian cells expressing a heterologous protein in a perfusion cell culture is provided comprising culturing mammalian cells expressing a heterologous protein in a perfusion culture medium comprising an effective amount of 100-600 µM arachidonic acid.

In a fourth aspect of the disclosure, a method of culturing mammalian cells expressing a heterologous protein in a perfusion cell culture is provided comprising culturing mammalian cells expressing a heterologous protein in a perfusion culture medium comprising an effective amount of 0.0001-100 µM prostaglandin E2.

Also described herein is a method of enhancing the specific productivity of a perfusion cell culture comprising (a) culturing recombinant mammalian cells encoding a protein of interest in a perfusion growth medium until a desired cell density is reached, and (b) introducing an effective amount of one or more of a lipid or lipid metabolite selected from the group consisting of: linoleic acid, arachidonic acid, and prostaglandin E2, or derivatives and/or precursors thereof, into the growth medium. In certain embodiments, the lipids or lipid metabolites or combination thereof results in growth suppression and/or increased cell specific productivity. In certain other embodiments relating to perfusion cell culture, the growth suppression and/or increased cell specific productivity may reduce or eliminate the need for cell bleeding to maintain or achieve a steady state.

In a fifth aspect of the disclosure, a perfusion cell culture medium for culturing mammalian cells and producing therapeutic proteins with improved productivity is provided. The perfusion cell culture medium comprises one or more of an effective amount of one or more lipids or lipid metabolites wherein the one or more lipids or lipid metabolites comprises 500-2000 µM linoleic acid, 100-600 µM arachidonic acid and/or 0.0001-100 µM prostaglandin E2, and wherein the perfusion cell culture medium is protein-free but optionally includes insulin and/or insulin-like growth factor. In certain embodiments, the lipids or lipid metabolites or combination thereof results in growth suppression and/or increased cell specific productivity. In certain other embodiments relating to perfusion cell culture, the growth suppression and/or increased cell specific productivity may reduce or eliminate the need for cell bleeding to maintain or achieve a steady state. The cell culture medium can be used in continuous cultures, particularly continuous perfusion cultures.

In various embodiments of the above aspects, the cell culture is not particularly limited and may encompass all forms and techniques of cell culture, including but not limited to, perfusion, continuous, finite, suspension, adherent or monolayer, anchorage-dependent, and 3D cultures. In a preferred embodiment, the cell culture is a continuous perfusion cell culture.

In various embodiments, the mammalian cells grown in cell culture are Chinese Hamster Ovary (CHO) cells. In various other embodiments, the mammalian cells are not particularly limited. They can include any commonly used cell lines from hamster (e.g., CHO or CHO-S cells), human (e.g., Jurkat cells, 293 cells, HeLa cells), monkey (e.g., CV-1 cells), or mouse (e.g., 3T3 cells). The cells can be from any tissue, for example ovary cells (e.g., CHO of CHO-S) or kidney cells (e.g., 293 cells). The cells used in any of the methods herein may also encompass any cells that may be obtained from commercial sources, e.g., THERMOFISHER SCIENTIFIC^{®}.

In various embodiments, the effective amount of linoleic acid is from about 1800-2000 µM. In one embodiment, the linoleic acid is in a concentration of 500-2000 µM.

In other embodiments, the effective amount of arachidonic acid is from about 150-500 µM. In one embodiment, the arachidonic acid is in a concentration of 100-600 µM.

In other embodiments, the effective amount of prostaglandin E2 is from about 0.001 µM to 60 µM. In still other embodiments, the effective amount of prostaglandin E2 is 0.0001 µM or more, 0.001 µM or more, 0.01 µM or more, 0.1 µM or more, 1 µM or more, 10 µM or more, 20 µM or more, 30 µM or more, 40 µM or more, 50 µM or more, 60 µM or more, 70 µM or more, 80 µM or more, 90 µM or more, wherein the upper limit of each range is 100 µM or less. In yet other embodiments, the effective amount of prostaglandin E2 is from about 1-100 µM, or from about 1-50 µM, or about 1-25 µM, or from about 0.0001-0.001 µM, to about 0.0005-0.01 µM, to about 0.005-0.1 µM, to about 0.05-1.0 µM to about 0.5-100 µM. In one embodiment, the prostaglandin E2 is in a concentration of 0.0001-100 µM.

In some embodiments, the culture medium comprises at least two of the lipids or lipid metabolites. In one embodiment, the culture medium comprises arachidonic acid at a concentration of 100-300 µM and linoleic acid at a concentration of 500-1800 µM. In another embodiment, the culture medium comprises prostaglandin E2 at a concentration of 0.0001-0.0009 µM in combination with either linoleic acid at a concentration of 500-1800 µM or arachidonic acid at a concentration of 100-150 µM.

In still another embodiment, the culture medium comprises all three lipids or lipid metabolites. In one embodiment, the culture medium comprises linoleic acid in a concentration of 500-2000 µM, the arachidonic acid at a concentration of 100-300 µM, and the prostaglandin E2 at a concentration of 0.0001-0.0009 µM.

In various embodiments, the heterologous protein is not particularly limited, and can be, for example, a therapeutic protein, such as, but not limited to an antibody, a fusion protein, a cytokine, or a growth factor, or a fragment thereof. The antibodies can be monoclonal antibodies and can be in any alternative molecular format, including (a) antigen-binding building blocks (e.g., single variable domain binding sites; two variable domain binding sites; diabody), (b) bispecific antibody fragments (e.g., tandem scFv, tandem scFv-Fc, scFv-Fc knobs-into-holes, scFv-Fc-scFv, F(ab')₂, Fab-scFv, (Fab'scFv)₂, diabody, scDiabody, scDiabody-Fc, scDiabody-C_{H}3), (c) IgG-based bispecific antibodies (e.g., hybrid hybridoma, knobs-into-holes with common light chain, two-in-one IgG, dual V domain IgG, IgG-scFv, scFv-IgG, IgG-V, and V-IgG). See Chan et al., Nature Reviews Immunology, Vol. 10, pp. 301-316 (May 2010).

In various embodiments, the one or more lipids or lipid metabolites are added or initiated only when the cells in the cell culture reach a certain density. In certain embodiments, the cell density is 10 x 10⁶ cells/ml to about 120 x 10⁶ cells/ml or even higher. The cell density for lipid initiation may also be at least 10 x 10⁶ cells/ml, at least 20 x 10⁶ cells/ml, at least 30 x 10⁶ cells/ml, at least 40 x 10⁶ cells/ml or at least 50 x 10⁶ cells.

In certain embodiments, a method is provided for culturing mammalian cells during a growth phase by perfusion with a serum-free perfusion medium until the cells reach a desired viable cell density; and then growing the cells during a production phase in a serum-free perfusion medium comprising one or more of the lipid or lipid metabolite selected from the group consisting of linoleic acid, arachidonic acid, and prostaglandin E2. In certain embodiments, the arachidonic acid, if present, is at a concentration of 100-300 µM, the linoleic acid, if present, is at a concentration of 500-1800 µM, and the prostaglandin E2, if present, is at a concentration of 0.0001-0.0009 µM. In certain other embodiments, the growth phase continues until the desired viable cell density (VCD) reaches 10 x 10⁶ cells/ml to about 120 x 10⁶ cells/ml or even higher, or reaches about 10 x 10⁶ cells/ml, or about 20 x 10⁶ cells/ml, or about 30 x 10⁶ cells/ml, or about 40 x 10⁶ cells/ml or about 50 x 10⁶ cells.

In certain embodiment pertaining to perfusion cell culture, the method of cell culture may include a further step of cell bleeding. However, the use of cell bleeding may be reduced or eliminated with the lipid-containing media of the herein described methods compared to a perfusion cell culture using the same perfusion medium but without including the lipids or lipid metabolites described herein.

The osmolarity of the perfusion medium can be in the range of between 300 and 1400 mOsmol/kg, preferably between 300 and 500 mOsmol/kg, more preferably between 330 and 450 mOsmol/kg and even more preferably between 360 and 390 mOsmol/kg.

The perfusion medium may be serum-free, and it may further be chemically defined and/or hydrolysate free. Preferably the perfusion medium can be protein-free or protein-free except for recombinant insulin and/or insulin-like growth factor, more preferably the serum-free perfusion medium is chemically defined and protein-free or protein-free except for recombinant insulin and/or insulin-like growth factor.

In a further aspect a method of producing a therapeutic protein using the methods of the invention is provided optionally comprising a further step of purifying and formulating the therapeutic protein into a pharmaceutically acceptable formulation.

In particular further embodiments, the present disclosure provides the following embodiments which are not in any way intended to limit the scope of the herewith disclosure.

In one embodiment, the disclosure provides a method of culturing mammalian cells expressing a heterologous protein in a perfusion cell culture, comprising: culturing mammalian cells expressing a heterologous protein in a culture medium comprising an effective amount of one or more lipids or lipid metabolites wherein the one or more lipids or lipid metabolites comprises 500-2000 µM linoleic acid, 100-600 µM arachidonic acid and/or 0.0001-100 µM prostaglandin E2. In certain embodiments, the lipids or lipid metabolites or combination thereof results in growth suppression and/or increased cell specific productivity. In certain other embodiments, the growth suppression and/or increased cell specific productivity may reduce or eliminate the need for cell bleeding to maintain or achieve a steady state.

In another embodiment, the invention provides a method of producing a therapeutic protein from a perfusion cell culture, comprising:
(a) culturing mammalian cells expressing a heterologous protein in a culture medium comprising an effective amount of one or more lipids or lipid metabolites wherein the one or more lipids or lipid metabolites comprises 500-2000 µM linoleic acid, 100-600 µM arachidonic acid and/or 0.0001-100 µM prostaglandin E2,
(b) harvesting the heterologous protein from the perfusion cell culture.

In certain embodiments, the lipids or lipid metabolites or combination thereof results in growth suppression and/or increased cell specific productivity. In certain other embodiments relating to perfusion cell culture, the growth suppression and/or increased cell specific productivity may reduce or eliminate the need for cell bleeding to maintain or achieve a steady state.

In various embodiments, the perfusion cell culture medium comprises one or more lipids or lipid metabolites selected from the group consisting of linoleic acid, arachidonic acid, and prostaglandin E2.

In various embodiments, the linoleic acid is in a concentration of 500-2000 µM.

In various embodiments, the arachidonic acid is in a concentration of 100-600 µM.

In various embodiments, the prostaglandin E2 is in a concentration of 0.0001-100 µM.

In various embodiments, the culture medium comprises two of the lipids or lipid metabolites.

In various embodiments, the culture medium comprises arachidonic acid at a concentration of 100-300 µM and linoleic acid at a concentration of 500-1800 µM.

In various embodiments, the culture medium comprises prostaglandin E2 at a concentration of 0.0001-0.0009 µM in combination with either linoleic acid at a concentration of 500-1800 µM or arachidonic acid at a concentration of 100-150 µM.

In various embodiments, the culture medium comprises all three lipids or lipid metabolites.

In various embodiments, the linoleic acid is in a concentration of 500-2000 µM, the arachidonic acid is at a concentration of 100-300 µM, and the prostaglandin E2 is at a concentration of 0.0001-0.0009 µM.

In various embodiments, the cell culture is a continuous perfusion cell culture.

In various embodiments, the mammalian cells are Chinese Hamster Ovary (CHO) cells, Jurkat cells, 293 cells, HeLa cells, CV-1 cells, or 3T3 cells, or a derivative of any of these cells, wherein said CHO cell can be further selected from the group consisting of a CHO-DG44 cell, a CHO-K1 cell, a CHO DXB11 cell, a CHO-S cell, and a CHO GS deficient cell or a derivative of any of these cells.

In various embodiments, the heterologous protein is a therapeutic protein, an antibody, or a therapeutically effective fragment thereof.

In various embodiments, the antibody is a monoclonal antibody or fragment thereof.

In various embodiments, the antibody is a bispecific antibody.

In various embodiments, the culture medium is a serum-free perfusion medium.

In various embodiments, the culture medium is optionally (a) chemically defined, (b) hydrolysate-free, or (c) protein-free but optionally includes insulin and/or insulin-like growth factor.

In various embodiments, the increased productivity is achieved when the total production of the heterologous protein produced by the cell culture is increased by at least 5%, or at least 6%, or at least 7%, or at least 8%, or at least 9%, or at least 10%, or at least 15%, or at least 20%, or at least 25%, or at least 30%, or at least 35%, or at least 40%, or at least 45%, or at least 50%, or at least 60%, or at least 70%, or at least 80%, or at least 90%, or at least 100%, or at least 200%, relative the level of total production in a control cell culture that does not include the lipids or lipid metabolites.

In various embodiments, the increased productivity is achieved when the cell specific productivity (pg/cell/day) of the cell culture is increased by at least 5%, or at least 6%, or at least 7%, or at least 8%, or at least 9%, or at least 10%, or at least 15%, or at least 20%, or at least 25%, or at least 30%, or at least 35%, or at least 40%, or at least 45%, or at least 50%, or at least 60%, or at least 70%, or at least 80%, or at least 90%, or at least 100%, or at least 200%, or at least 5-10%, or at least 7.5-20%, or at least 10-30%, or at least 12.5-40%, or at least 15-50%, or at least 5-50% relative the cell specific productivity in a control cell culture that does not include the lipids or lipid metabolites.

In various embodiments, the growth suppression is sufficient to maintain the cells in a steady state having a viable cell density that is at least 50% lower, at least 40% lower, at least 30% lower, at least 20% lower, at least 15% lower, or at least 10% lower, or at least 5% lower, or at least 2.5% lower, or 1% or lower relative a control cell culture that does not include the lipids or lipid metabolites.

In various embodiments, the cell culture on day 2 is changed to a perfusion cell culture.

In various embodiments, the perfusion rate increases after perfusion has started.

In various embodiments, the perfusion rate increases from less or equal to 0.5 vessel volumes per day to 5 vessel volumes per day.

In various embodiments, the perfusion rate increases from less or equal to 0.5 vessel volumes per day to 2 vessel volumes per day.

In various embodiments, the method further comprises harvesting the heterologous protein from the cell culture in a continuous manner.

In various embodiments, the one or more lipids or lipid metabolites are added to the cell medium once a cell density of 10 x 10⁶ cells/ml to about 120 x 10⁶ cells/ml is reached. In one embodiment, the methods and/or growth media disclosed herein comprises 500 µM of linoleic acid, which decreases the VCD by 6-10% relative to cell culture that does not comprise linoleic acid in its growth medium.

In another embodiment, the methods and/or growth media disclosed herein comprises 900 µM of linoleic acid, which decreases the VCD by 11-14% relative to cell culture that does not comprise linoleic acid in its growth medium.

In still another embodiment, the methods and/or growth media disclosed herein comprises 1350 µM of linoleic acid, which decreases the VCD by 11-25% relative to cell culture that does not comprise linoleic acid in its growth medium.

In one embodiment, the methods and/or growth media disclosed herein comprises 1800 µM of linoleic acid, which decreases the VCD by 23-39.8% relative to cell culture that does not comprise linoleic acid in its growth medium.

In another embodiment, the methods and/or growth media disclosed herein comprises 500 µM of arachidonic acid, which decreases the VCD by 15-36% relative to cell culture that does not comprise arachidonic acid in its growth medium.

In another embodiment, the methods and/or growth media disclosed herein comprises 0.001 µM of prostaglandin E2, which decreases the VCD by 17% relative to cell culture that does not comprise arachidonic acid in its growth medium.

In another embodiment, the methods and/or growth media disclosed herein comprises 10 µM of prostaglandin E2, which decreases the VCD by 28% relative to cell culture that does not comprise arachidonic acid in its growth medium.

In another embodiment, the methods and/or growth media disclosed herein comprises 20 µM of prostaglandin E2, which decreases the VCD by 39% relative to cell culture that does not comprise arachidonic acid in its growth medium.

In another embodiment, the methods and/or growth media disclosed herein comprises 60 µM of prostaglandin E2, which decreases the VCD by 30% relative to cell culture that does not comprise arachidonic acid in its growth medium.

In another embodiment, the methods and/or growth media disclosed herein comprises 10 µM of prostaglandin E2, which decreases the VCD by 28% relative to cell culture that does not comprise arachidonic acid in its growth medium.

In one embodiment, the methods and/or growth media disclosed herein comprises 500 µM of linoleic acid, which increases the cell specific productivity by up to 10% relative to cell culture that does not comprise linoleic acid in its growth medium.

In one embodiment, the methods and/or growth media disclosed herein comprises 900 µM of linoleic acid, which increases the cell specific productivity by up to 14% relative to cell culture that does not comprise linoleic acid in its growth medium.

In one embodiment, the methods and/or growth media disclosed herein comprises 1350 µM of linoleic acid, which increases the cell specific productivity by up to 14% relative to cell culture that does not comprise linoleic acid in its growth medium.

In one embodiment, the methods and/or growth media disclosed herein comprises 1800 µM of linoleic acid, which increases the cell specific productivity by up to 30% relative to cell culture that does not comprise linoleic acid in its growth medium.

In one embodiment, the methods and/or growth media disclosed herein comprises 500 µM of arachidonic acid, which increases the cell specific productivity by up to 52% relative to cell culture that does not comprise arachidonic acid in its growth medium.

In one embodiment, the methods and/or growth media disclosed herein comprises 0.001 µM of prostaglandin E2, which increases the cell specific productivity by up to 13.5% relative to cell culture that does not comprise prostaglandin E2 in its growth medium.

In one embodiment, the methods and/or growth media disclosed herein comprises 10 µM of prostaglandin E2, which increases the cell specific productivity by up to 34.4% relative to cell culture that does not comprise prostaglandin E2 in its growth medium.

In one embodiment, the methods and/or growth media disclosed herein comprises 20 µM of prostaglandin E2, which increases the cell specific productivity by up to 31% relative to cell culture that does not comprise prostaglandin E2 in its growth medium.

In one embodiment, the methods and/or growth media disclosed herein comprises 60 µM of prostaglandin E2, which increases the cell specific productivity by up to 31% relative to cell culture that does not comprise prostaglandin E2 in its growth medium.

### BRIEF DESCRIPTION OF THE DRAWINGS

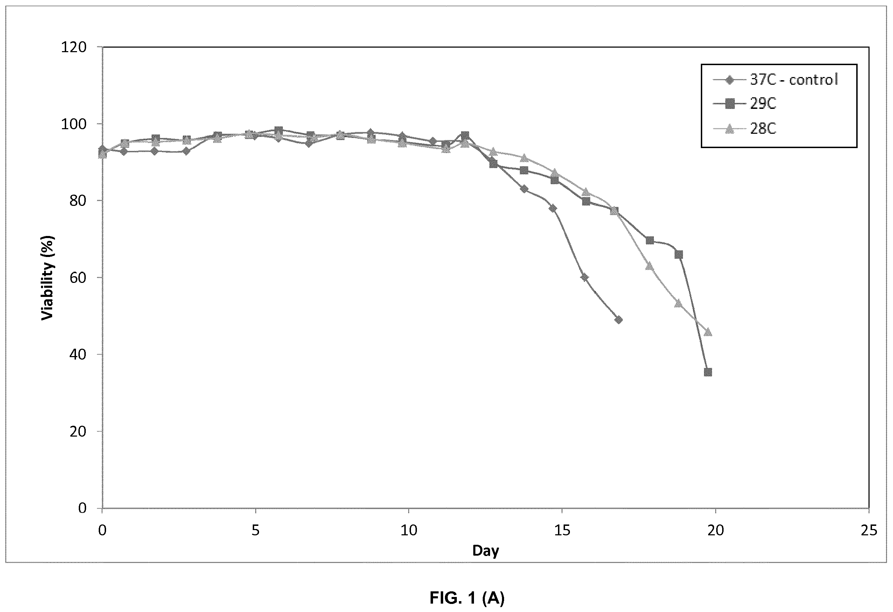

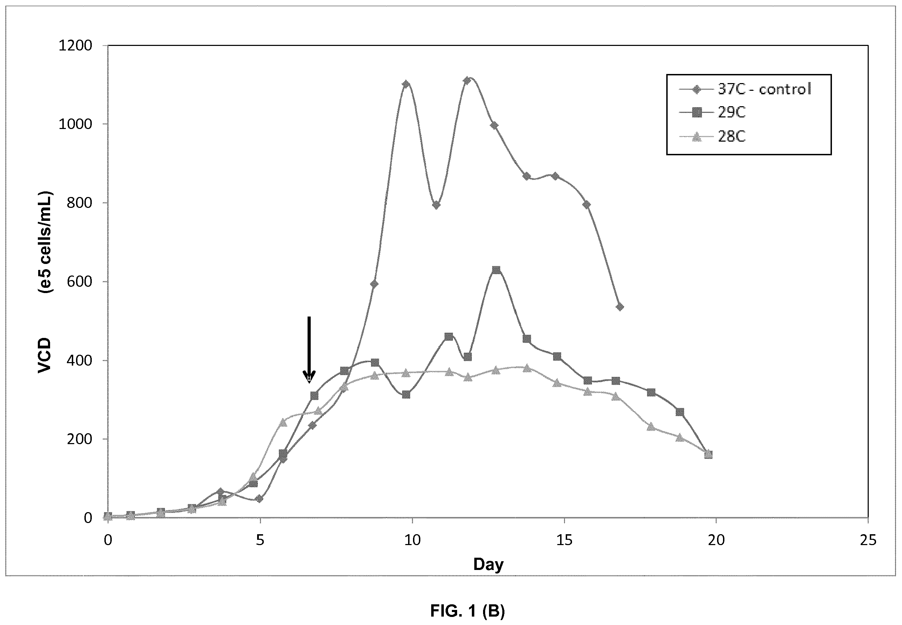

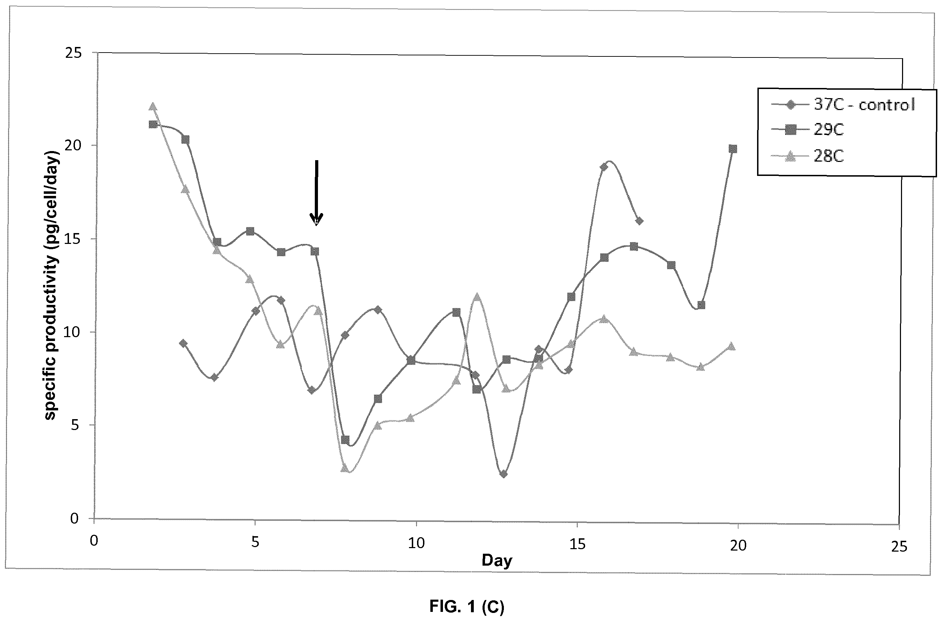

**FIG. 1****.** The effect of a temperature shift on (A) % percent viability, (B) viable cell density (e⁵ cells/mL), and (C) specific productivity (pg/cell/day) of CHO cell line expressing a heterologous protein in perfusion cell culture. Perfusion was started on day 2 of cell culture and gradually was ramped up to an exchange of 2 vessel volumes per day (2VVD). The arrow indicates the day when the temperature was shifted from 37°C to the indicated value in the legend (29°C - squares- or 28°C -triangles-). Further details can be found in Example 1.

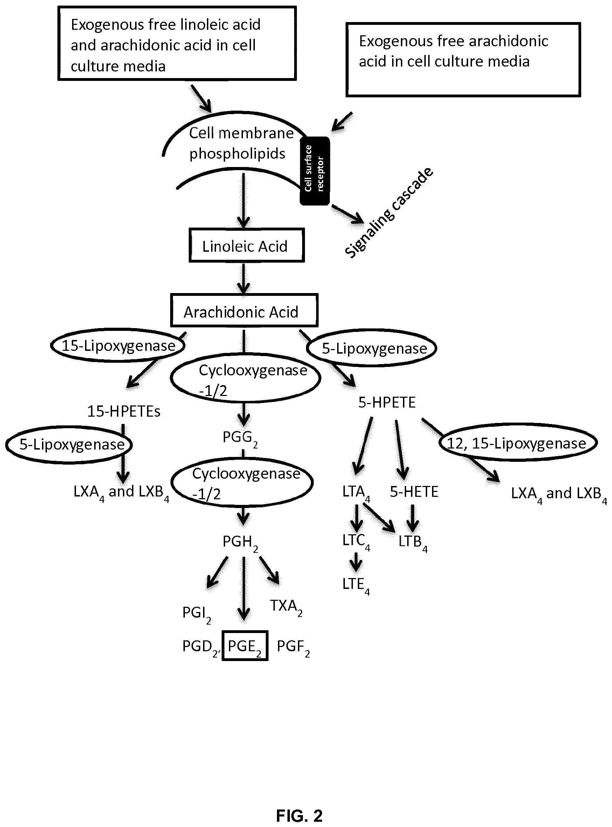

**FIG. 2****.** Depicts the metabolic pathway of linoleic acid metabolism. High concentrations of exogenous free linoleic acid are shown to diffuse through the cell membrane which is then converted to arachidonic acid. The arachidonic acid is then metabolized through a multi-step reaction to PGE₂, among other downstream metabolites.

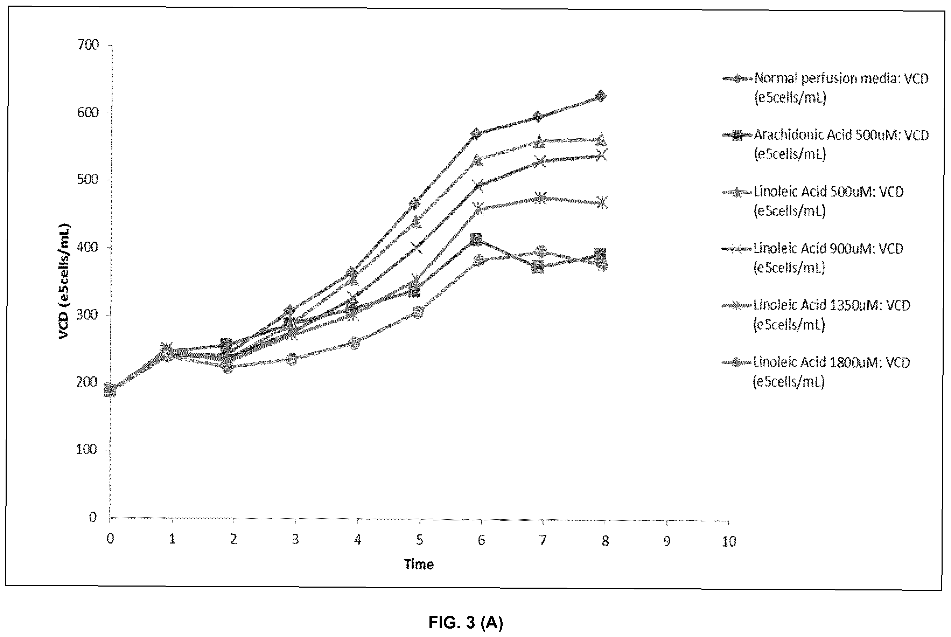

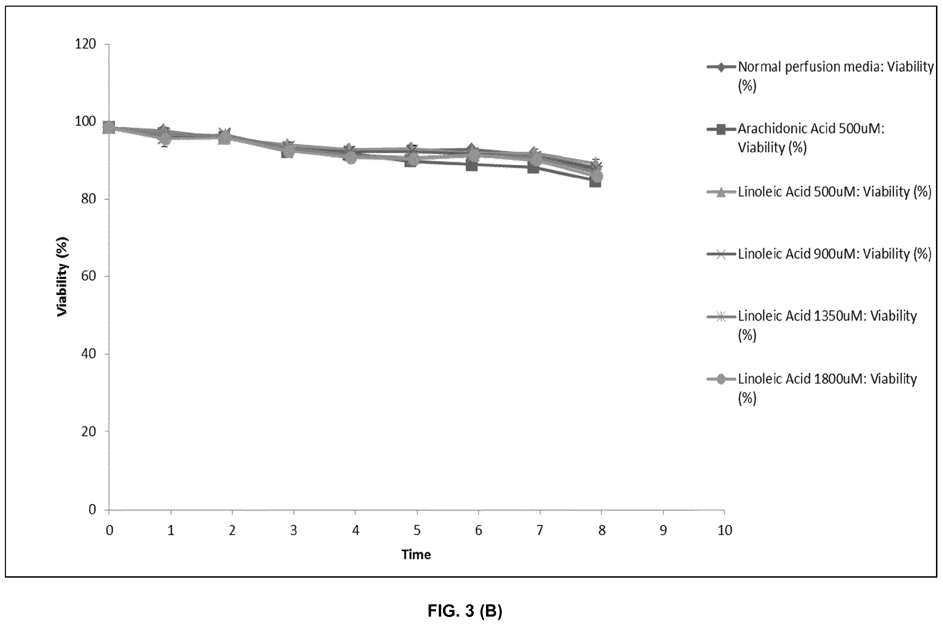

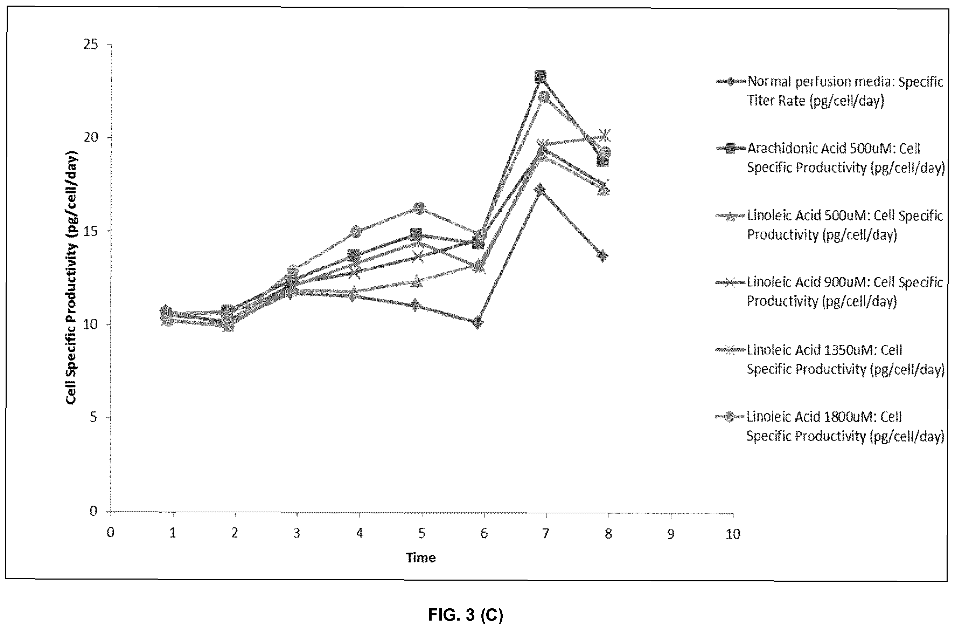

**FIG. 3****.** Demonstrates that linoleic acid when added to perfusion medium suppresses cell growth and increases cell specific productivity (qₚ) in CHO perfusion cell culture. Linoleic acid at various concentrations 500 µM, 900 µM, 1350 µM and 1800 µM was added to the cell culture media and the effects on (A) viable cell density (e⁵ cells/ml), (B) percent (%) cell viability and (C) specific productivity (qₚ) were determined. Further details can be found in Example 2.

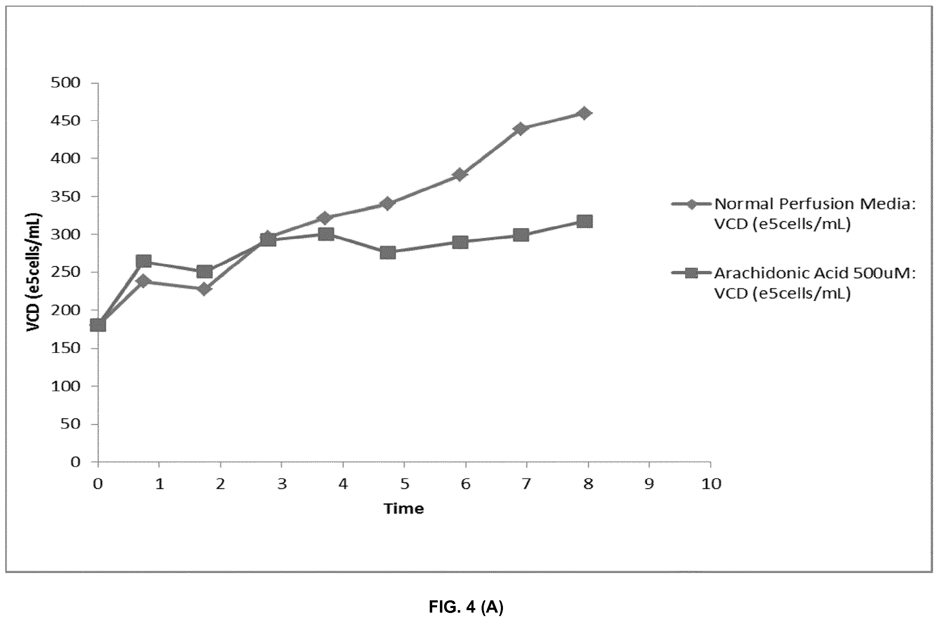

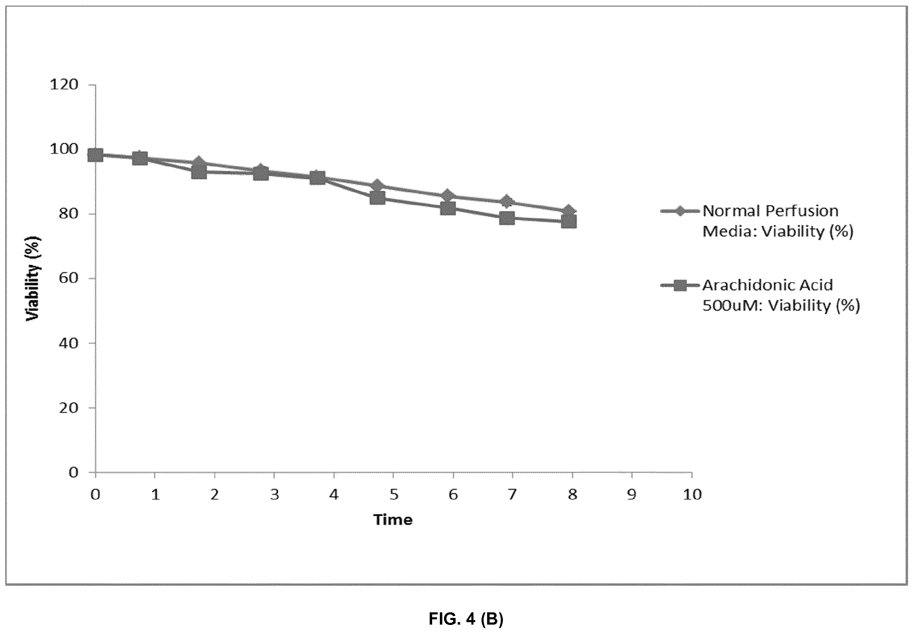

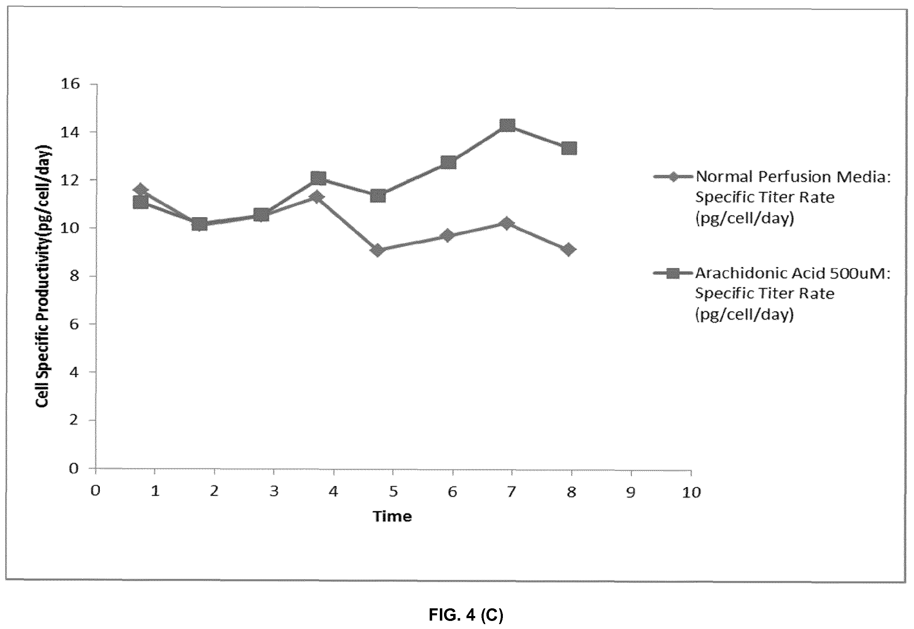

**FIG. 4****.** Demonstrates that arachidonic acid when added to perfusion medium suppresses cell growth and increases cell specific productivity (qₚ). The effect of arachidonic acid on (A) viable cell density (e⁵ cells/ml), (B) percent (%) cell viability and (C) specific productivity (qₚ) of the cell culture was determined. Arachidonic concentration at 500 µM added in the normal perfusion media suppressed cell growth up to 31% and increased cell specific productivity by 46% or higher. Further details can be found in Example 3.

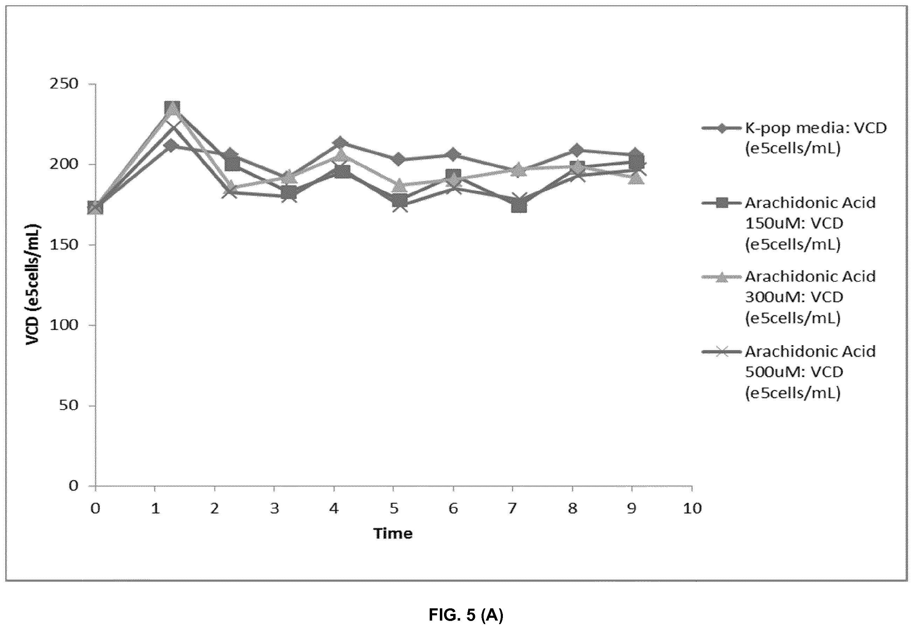

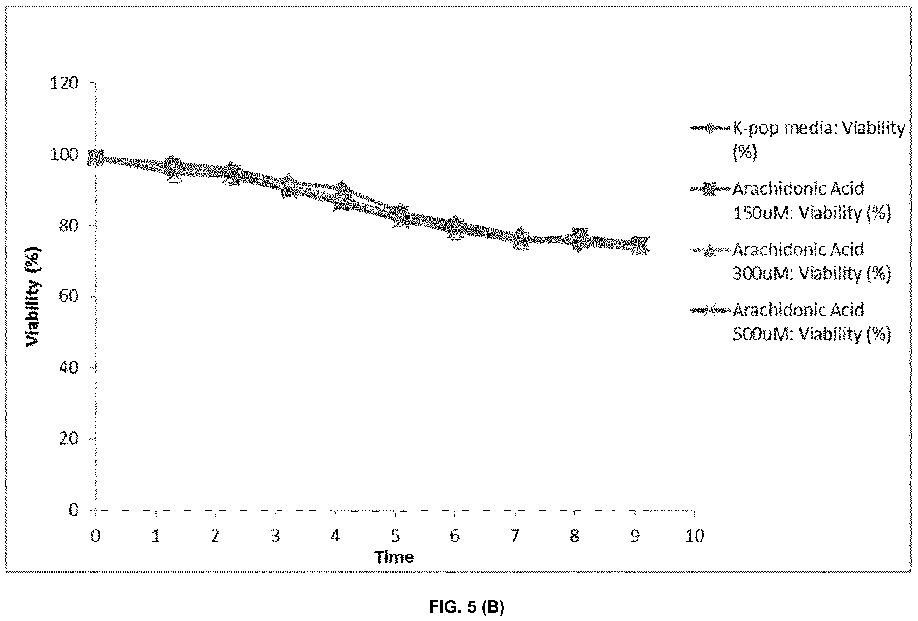

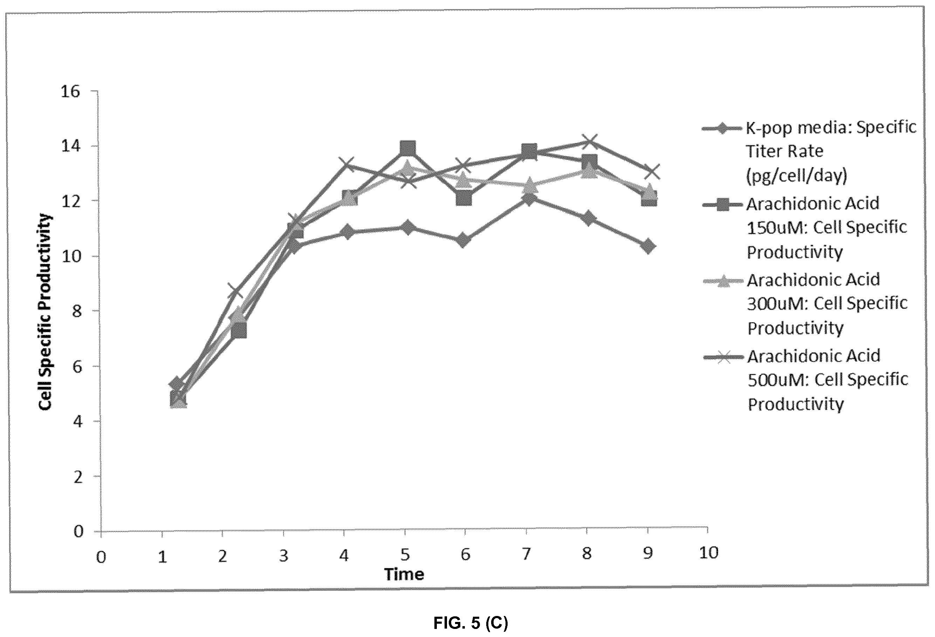

**FIG. 5****.** Demonstrates that arachidonic when added to perfusion "k-pop" medium suppresses cell growth and increases cell specific productivity (qₚ). The k-pop medium in this instance has a sodium concentration of about 34 mM and a raised potassium concentration of approximately 94 mM, i.e., a reduced sodium-to-potassium ratio of 0.4 mol Na⁺/mol K⁺. The effect of concentrations of arachidonic acid at 150 µM, 300 µM and 500 µM in a k-pop baseline with regard to (A) viable cell density (e⁵ cells/ml), (B) percent (%) cell viability and (C) specific productivity (qₚ) was determined. Further details can be found in Example 4.

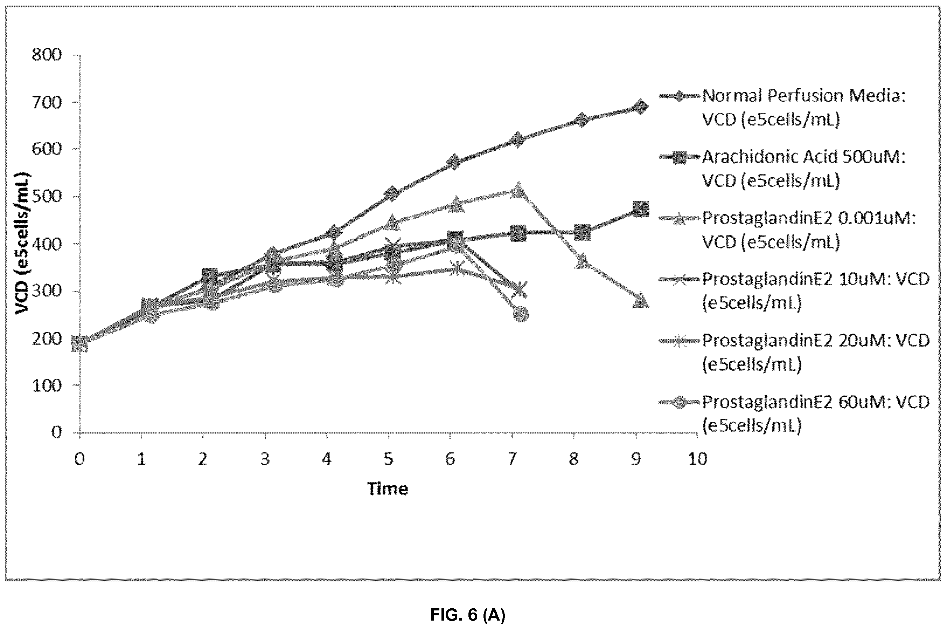

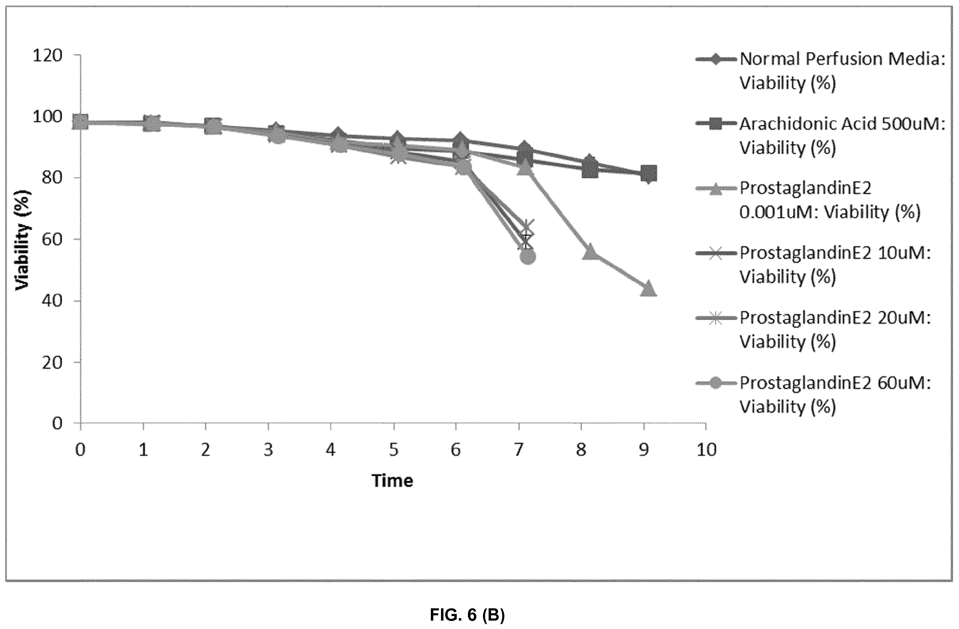

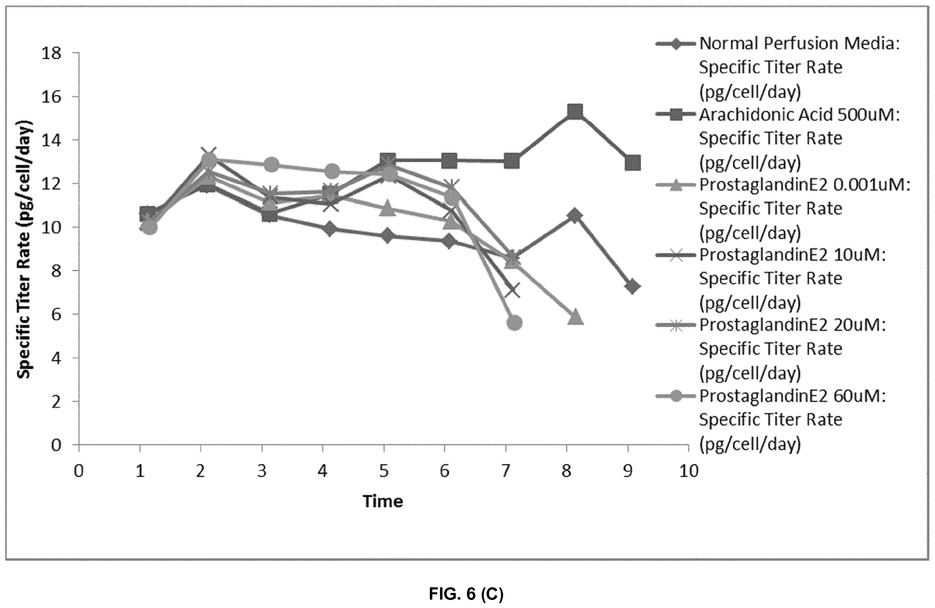

**FIG. 6****.** Demonstrates that prostaglandin E2 when added to perfusion medium suppresses cell growth and increases cell specific productivity (qₚ). FIG. 6 demonstrates the effects of the concentration of arachidonic acid at 500 µM, PGE2 0.001 µM, 10 µM, 20 µM and 60 µM added to the perfusion media on (A) viable cell density (VCD) (e5 cells/ml), (B) percent of cell viability, and (C) specific productivity (pg/cell/day). Further details can be found in Example 5.

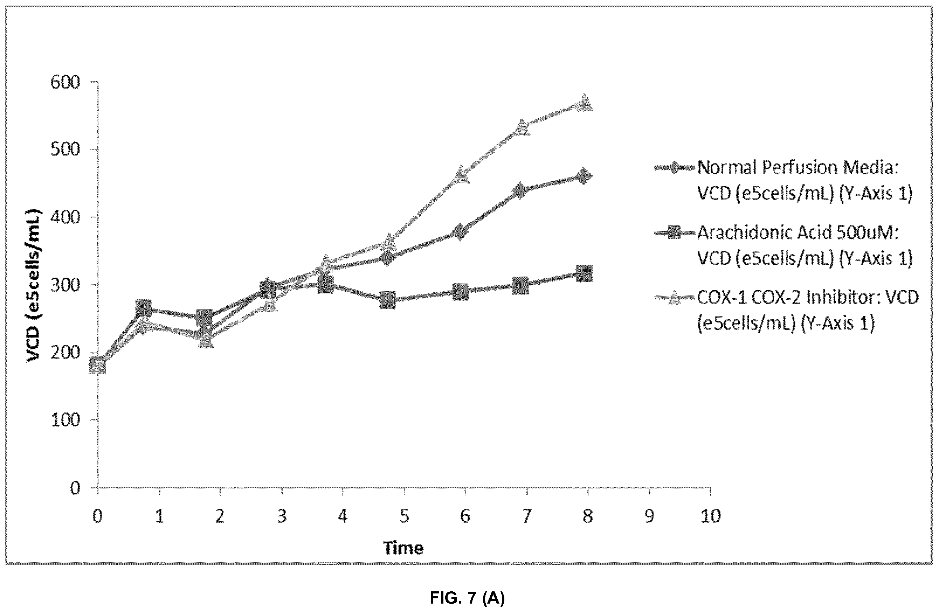

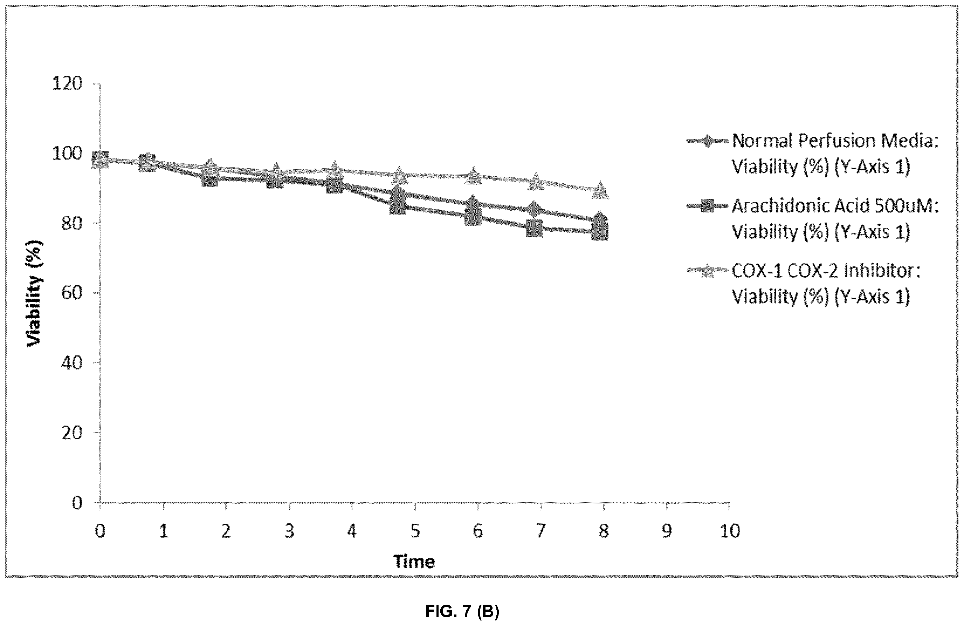

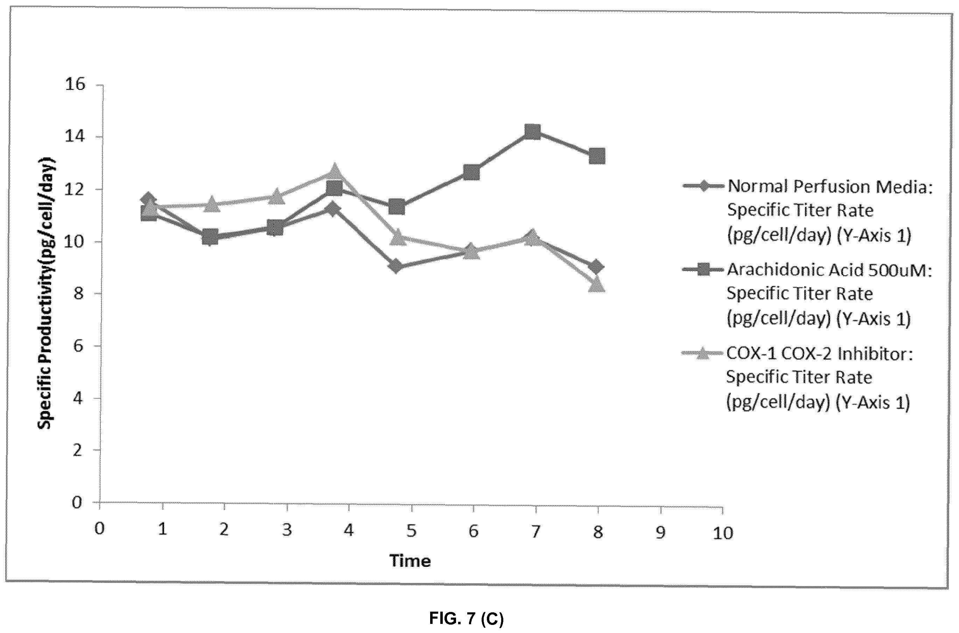

**FIG. 7****.** Demonstrates the effect of acetylsalicylic acid (ASA) on (A) viable cell density, (B) percent viability, and (C) specific productivity in perfusion media with 500 µM of arachidonic acid. Further details can be found in Example 6.

### DETAILED DESCRIPTION

The present invention relates in part to the discovery that certain lipids and lipid metabolites when included singularly or in combination in perfusion cell culture media, e.g., linoleic acid, arachidonic acid, and prostaglandin E2, are effective at causing an increase in viability and cell specific productivity of the cell culture. These agents can be used in connection with any type of cell culture system, including continuous perfusion cell culture systems. In the case of perfusion cell culture, the use of the lipids and/or lipid metabolites can reduce or eliminate the need for cell bleeding as a means for maintaining cell culture steady state, i.e., the lipids and/or lipid metabolites alone are capable of maintaining or sustaining cell culture steady state by decreasing viable cell density and suppressing cell growth.

Without being bound by theory, and as demonstrated in the Examples, prostaglandin E2 is a central key factor which triggers cell growth suppression and the increased cell specific productivity. Linoleic acid and arachidonic acid are precursors of prostaglandin E2 synthesis and are part of the same metabolic pathway (see FIG. 2). Thus, described herein is a wide spectrum of approaches to effectively by direct or indirect means increase the intracellular concentration of prostaglandin E2, including, but not limited to adding exogenous prostaglandin E2 to the cell culture media and adding exogenous prostaglandin E2 metabolic precursors to the cell culture media, including linoleic acid and/or arachidonic acid.

### Definitions

Definitions of certain terms are provided below. In general, any terms presented in this disclosure should be given their ordinary meaning in the art, unless otherwise stated or defined.

The general embodiments "comprising" or "comprised" encompass the more specific embodiment "consisting of". Furthermore, singular and plural forms are not used in a limiting way. As used herein, the singular forms "a", "an" and "the" designate both the singular and the plural, unless expressly stated to designate the singular only.

The term "perfusion" as used herein refers to maintaining a cell culture bioreactor in which equivalent volumes of media are simultaneously added and removed from the reactor while the cells are retained in the reactor. A perfusion culture may also be referred to as continuous culture. This provides a steady source of fresh nutrients and constant removal of cell waste products. Perfusion is commonly used to attain much higher cell density and thus a higher volumetric productivity than conventional bioreactor batch or fed batch conditions. Secreted protein products can be continuously harvested while retaining the cells in the reactor, e.g., by filtration, alternating tangential flow (ATF), cell sedimentation, ultrasonic separation, hydrocyclones, or any other method known to the person skilled in the art or as described Kompala and Ozturk (Cell Culture Technology for Pharmaceutical and Cell-Based Therapies, (2006), Taylor & Francis Group, LLC, pages 387-416). Mammalian cells may be grown in suspension cultures (homogeneous cultures) or attached to surfaces or entrapped in different devices (heterogeneous cultures). In order to keep the working volume in the bioreactor constant the harvest rate and cell bleed (fluid removal) should be equal to the predetermined perfusion rate. The culture is typically initiated by a batch culture and the perfusion is started on day 2-3 after inoculation when the cells are still in exponential growth phase and before nutrient limitation occurs.

Perfusion based methods offer potential improvement over the batch and fed-batch methods by adding fresh media and simultaneously removing spent media. Large scale commercial cell culture strategies may reach high cell densities of 60 - 90 x 10⁶ cells/mL, at which point about a third to over half of the reactor volume may be biomass. With perfusion based culture, extreme cell densities of >1 x 10⁸ cells/mL have been achieved. Typical perfusion cultures begin with a batch culture start-up lasting for a day or more to enable rapid initial cell growth and biomass accumulation, followed by continuous, step-wise and/or intermittent addition of fresh feed media to the culture and simultaneous removal of spent media with retention of cells throughout the growth and production phases of the culture. Various methods, such as sedimentation, centrifugation, or filtration, can be used to remove spent media, while maintaining the cells. Perfusion flow rates of a fraction of a working volume per day up to many multiple working volumes per day have been utilized.

The term "perfusion rate" as used herein is the volume added and removed and is typically measured per day. It depends on the cell density and the medium. It should be minimized to reduce the dilution of the product of interest, i.e., harvest titer, while ensuring adequate rates of nutrient addition and by-product removal. Perfusion is typically started on day 2-3 after inoculation when the cells are still in the exponential growth phase and hence perfusion rate may be increased over the culture. Increase in perfusion rate may be incremental or continuously, i.e., based on cell density or nutrient consumption. It typically starts with 0.5 or 1 vessel volume per day (VVD) and may go up to about 5 VVD. Preferably, the perfusion rate is between 0.5 to 2 VVD. The increase may be by 0.5 to 1 VVD. For continuous increase in perfusion, a biomass probe may be interfaced with the harvest pump, such that the perfusion rate is increased as a linear function of the cell density determined by the biomass probe, based on a desired cell specific perfusion rate (CSPR). The CSPR equals the perfusion rate per cell density and an ideal CSPR depend on the cell line and the cell medium. The ideal CSPR should result in optimal growth rate and productivity. A CSPR of 50 to 100 pL/cell per day may be a reasonable starting range, which can be adjusted to find the optimal rate for a specific cell line.

The term "steady state" as used herein refers to the condition where cell density and bioreactor environment remain relatively constant. This can be achieved by cell bleeding, nutrient limitation and/or temperature reduction. In most perfusion cultures nutrient supply and waste removal will allow for constant cell growth and productivity and cell bleeding is required to maintain a constant viable cell density or to maintain the cells in steady state. A typical viable cell density at steady state is 10 to 50e6 cells/ml. The viable cell density may vary depending on the perfusion rate. A higher cell density can be reached by increasing the perfusion rate or by optimizing the medium for use with perfusion. At a very high viable cell density perfusion cultures become difficult to control within a bioreactor.

The terms "cell bleed" and "cell bleeding" are used interchangeably herein and refer to the removal of cells and medium from the bioreactor in order to maintain a constant, sustainable viable cell density within the bioreactor. The constant, sustainable viable cell density may also be referred to as target cell density. This cell bleed may be done using a dip tube and a peristaltic pump at a defined flow rate. The tubing should have the right size with a too narrow tube being prone to cell aggregation and clogging while if too large the cells may settle. The cell bleed can be determined based on growth rate, thus viable cell density can be limited to a desired volume in a continuous manner. Alternatively, cells may be removed at a certain frequency, e.g., once a day, and replaced by media to maintain cell density within a predictable range. Ideally the cell bleed rate is equal to the growth rate to maintain a steady cell density.

Typically, the product of interest removed with the cell bleed is discarded and therefore lost for the harvest. Opposite to a permeate, the cell bleed contains cells, which makes storage of the product prior to purification more difficult and can have detrimental effects on product quality. Thus, the cells would have to be removed continuously prior to storage and product purification, which would be laborious and cost inefficient. For slow growing cells the cell bleed may be about 10% of the removed fluid and for fast growing cells the cell bleed may be about 30% of the removed fluid. Thus, the product loss through the cell bleed may be about 30% of the product produced in total. The "permeate" as used herein refers to the harvest from which the cells have been separated to be retained in the culture vessel.

The term "culture" or "cell culture" is used interchangeably and refer to a cell population that is maintained in a medium under conditions suitable to allow survival and/or growth of the cell population. The present invention only relates to mammalian cell cultures and perfusion cell culture. Mammalian cells may be cultured in suspension or while attached to a solid support. As will be clear to the person skilled in the art, a cell culture refers to a combination comprising the cell population and the medium in which the population is suspended. The particular type of cell culture may encompass all forms and techniques of cell culture, including but not limited to, perfusion, continuous, finite, suspension, adherent or monolayer, anchorage-dependent, and 3D cultures.

The term "culturing" as used herein refers to a process by which mammalian cells are grown or maintained under controlled conditions and under conditions that supports growth and/or survival of the cells. The term "maintaining cells" as used herein is used interchangeably with "culturing cells". Culturing may also refer to a step of inoculating cells in a culture medium.

As used herein, the term "batch culture" is a discontinuous method where cells are grown in a fixed volume of culture media for a short period of time followed by a full harvest. Cultures grown using the batch method experience an increase in cell density until a maximum cell density is reached, followed by a decline in viable cell density as the media components are consumed and levels of metabolic by-products (such as lactate and ammonia) accumulate. Harvest typically occurs at or soon after the point when the maximum cell density is achieved (typically 5-10 x 10⁶ cells/mL, depending on media formulation, cell line, etc.) typically around 3 to 7 days.

As used herein, the term "fed-batch culture" improves on the batch process by providing bolus or continuous media feeds to replenish those media components that have been consumed. Since fed-batch cultures receive additional nutrients throughout the culture process, they have the potential to achieve higher cell densities (>10 to 30 x 10⁶ cells/ml, depending on media formulation, cell line, etc.) and increased product titers, when compared to the batch method. Unlike the batch process, a biphasic culture can be created and sustained by manipulating feeding strategies and media formulations to distinguish the period of cell proliferation to achieve a desired cell density (the growth phase) from the period of suspended or slow cell growth (the production phase). As such, fed batch cultures have the potential to achieve higher product titers compared to batch cultures. As with the batch method, metabolic by-product accumulation will lead to declining cell viability over time as these progressively accumulate within the cell culture media, which limits the duration of the production phase to about 1.5 to 3 weeks. Fed-batch cultures are discontinuous and harvest typically occurs when metabolic by-product levels or the culture viability reach predetermined levels.

The term "polypeptide" or "protein" is used interchangeably herein with "amino acid residue sequences" and refers to a polymer of amino acids. These terms also include proteins that are post-translationally modified through reactions that include, but are not limited to, glycosylation, acetylation, phosphorylation or protein processing. Modifications and changes, for example fusions to other proteins, amino acid sequence substitutions, deletions or insertions, can be made in the structure of a polypeptide while the molecule maintains its biological functional activity. For example certain amino acid sequence substitutions can be made in a polypeptide or its underlying nucleic acid coding sequence and a protein can be obtained with the same properties. The terms also apply to amino acid polymers in which one or more amino acid residue is an analog or mimetic of a corresponding naturally occurring amino acid. The term "polypeptide" typically refers to a sequence with more than 10 amino acids and the term "peptide" to sequences with up to 10 amino acids in length.

The term "heterologous protein" as used herein refers to a polypeptide derived from a different organism or a different species from the host cell. The heterologous protein is coded for by a heterologous polynucleotide that is experimentally put into the host cell that does not naturally express that protein. A heterologous polynucleotide may also be referred to as transgene. Thus, it may be a gene or open reading frame (ORF) coding for a heterologous protein. The term "heterologous" when used with reference to a protein may also indicate that the protein comprises amino acid sequences that are not found in the same relationship to each other or the same length in nature. Thus, it also encompasses recombinant proteins. Heterologous may also refer to a polynucleotide sequence, such as a gene or transgene, or a portion thereof, being inserted into the mammalian cell's genome in a location in which it is not typically found. In the present invention the heterologous protein is preferably a therapeutic protein.

The term "medium", "cell culture medium" and "culture medium" are used interchangeably herein and refer to a solution of nutrients that nourish cells, particularly mammalian cells. Cell culture media formulations are well known in the art. Typically a cell culture medium provides essential and non-essential amino acids, vitamins, energy sources, lipids and trace elements required by the cell for minimal growth and/or survival, as well as buffers, and salts. A culture medium may also contain supplementary components that enhance growth and/or survival above the minimal rate, including, but not limited to, hormones and/or other growth factors, particular ions (such as sodium, chloride, calcium, magnesium, and phosphate), buffers, vitamins, nucleosides or nucleotides, trace elements (inorganic compounds usually present at very low final concentrations), amino acids, lipids, and/or glucose or other energy source; as described herein. In certain embodiments, a medium is advantageously formulated to a pH and salt concentration optimal for cell survival and proliferation. The medium according to the invention is a perfusion culture medium that is added after the beginning of the cell culture. In certain embodiments, the cell culture medium is a mixture of a starting nutrient solution (basal medium or inoculation medium) and any culture medium that is added after the beginning of the cell culture. Some cell culture systems, e.g., perfusion cell culture, may have different phases of culturing, including a growth phase and a production phase. The particular medium used during growth phase and production phase may be particularly designed for implementation in said specific phase. In some embodiments, the lipids and/or lipid metabolites may be added or included in production phase only, or growth phase only, or both in growth phase and production phase.

The term "serum-free" as used herein refers to a cell culture medium that does not contain animal or human serum, such as fetal bovine serum. Preferably serum-free medium is free of proteins isolated from any animal or human derived serum. Various tissue culture media, including defined culture media, are commercially available, for example, any one or a combination of the following cell culture media can be used: RPMI-1640 Medium, RPMI-1641 Medium, Dulbecco's Modified Eagle's Medium (DMEM), Minimum Essential Medium Eagle, F-12K Medium, Ham's F12 Medium, Iscove's Modified Dulbecco's Medium, McCoy's 5A Medium, Leibovitz's L-15 Medium, and serum- free media such as EX-CELL^{™} 300 Series (JRH Biosciences, Lenexa, Kansas), among others. Serum-free versions of such culture media are also available. Cell culture media may be supplemented with additional or increased concentrations of components such as amino acids, salts, sugars, vitamins, hormones, growth factors, buffers, antibiotics, lipids, trace elements and the like, depending on the requirements of the cells to be cultured and/or the desired cell culture parameters.

The term "protein-free" as used herein refers to a cell culture medium that does not contain any protein. Thus, it is devoid of proteins isolated from an animal or human, derived from serum or recombinantly produced proteins, such as recombinant proteins produced in mammalian, bacterial, insect or yeast cells. A protein-free medium may contain single recombinant proteins, such as insulin or insulin-like growth factor, but only if this addition is explicitly stated.

As used herein the term "chemically defined" refers to a culture medium, which is serum-free and which does not contain any hydrolysates, such as protein hydrolysates derived from yeast, plants or animals. Preferably a chemically defined medium is also protein-free or contains only selected recombinantly produced (not animal derived) proteins, such as insulin or insulin-like growth factor. Chemically defined medium consist of a mixture of characterized and purified substances. An example of a chemically defined medium is for example CD-CHO medium from Invitrogen (Carlsbad, CA, US).

The term "suspension cells" or "non-adherent cells" as used herein relates to cells that are cultured in suspension in liquid medium. Adhesive cells such as CHO cells may be adapted to be grown in suspension and thereby lose their ability to attach to the surface of the vessel or tissue culture dish.

As used herein, the term "bioreactor" means any vessel useful for the growth of a cell culture. A bioreactor can be of any size as long as it is useful for the culturing of cells; typically, a bioreactor is sized appropriate to the volume of cell culture being grown inside of it. Typically, a bioreactor will be at least 1 liter and may be 2, 5, 10, 50, 100, 200, 250, 500, 1,000, 1,500, 2,000, 2,500, 5,000, 8,000, 10,000, 12,000 liters or more, or any volume in between. The internal conditions of the bioreactor, including, but not limited to pH and temperature, can be controlled during the culturing period. Those of ordinary skill in the art will be aware of, and will be able to select, suitable bioreactors for use in practicing the present invention based on the relevant considerations. The cell cultures used in the methods of the present invention can be grown in any bioreactor suitable for perfusion culture. The particular type of bioreactor is not particularly limited and may encompass all types of bioreactors, including but not limited to, fed-batch, batch, perfusion, continuous, finite, suspension, adherent or monolayer, anchorage-dependent, and 3D cultures.

As used herein, "cell density" refers to the number of cells in a given volume of culture medium. "Viable cell density" refers to the number of live cells in a given volume of culture medium, as determined by standard viability assays (such as trypan blue dye exclusion method).

As used herein, the term "cell viability" means the ability of cells in culture to survive under a given set of culture conditions or experimental variations. The term as used herein also refers to that portion of cells which are alive at a particular time in relation to the total number of cells, living and dead, in the culture at that time.

As used herein, the term "titer" means the total amount of a polypeptide or protein of interest (which may be a naturally occurring or recombinant protein of interest) produced by a cell culture in a given amount of medium volume. Titer can be expressed in units of milligrams or micrograms of polypeptide or protein per milliliter (or other measure of volume) of medium.

As used herein, the term "yield" refers to the amount of heterologous protein produced in perfusion culture over a certain period of time. The "total yield" refers to the amount of heterologous protein produced in perfusion culture over the entire run.

The term "reduction", "reduced," or "reduce," as used herein, generally means a decrease by at least 10% as compared to a reference level, for example a decrease by at least about 20%, or at least about 30%, or at least about 40%, or at least about 50%, or at least about 60%, or at least about 70%, or at least about 75%, or at least about 80%, or at least about 90% or up to and including a 100% decrease, or any integer decrease between 10-100% as compared to a control mammalian cell culture, which is cultured under the same conditions using the same serum-free perfusion medium without the lipids or lipid metabolites at the concentrations used in the perfusion medium of the invention.

The term "enhancement", "enhanced", "increase", or "increased", as used herein, generally means an increase by at least 10% as compared to a control cell, for example an increase by at least about 20%, or at least about 30%, or at least about 40%, or at least about 50%, or at least about 75%, or at least about 80%, or at least about 90%, or at least about 100%, or at least about 200%, or at least about 300%, or any integer decrease between 10-300% as compared to a mammalian cell culture, which is cultured under the same conditions using the same serum-free perfusion medium without the lipids or lipid metabolites at the concentrations used in the perfusion medium of the invention.

As used herein, a "control cell culture" or "control mammalian cell culture" is a cell which is the same as the cell culture to which it is compared to, except that the perfusion medium does not have the lipids or lipid metabolites at the concentrations used in the perfusion medium of the invention.

The term "mammalian cells" as used herein are cells lines suitable for the production of a heterologous protein, preferably a therapeutic protein, more preferably a secreted recombinant therapeutic protein. Preferred mammalian cells according to the invention are rodent cells such as hamster cells. The mammalian cells are isolated cells or cell lines. The mammalian cells are preferably transformed and/or immortalized cell lines. They are adapted to serial passages in cell culture and do not include primary non-transformed cells or cells that are part of an organ structure. Preferred mammalian cells are BHK21, BHK TK-, CHO, CHO-K1, CHO-S cells, CHO-DXB11 (also referred to as CHO-DUKX or DuxB11), and CHO-DG44 cells or the derivatives/progenies of any of such cell line. Particularly preferred are CHO-DG44, CHO-K1 and BHK21, and even more preferred are CHO-DG44 and CHO-K1 cells. Most preferred are CHO-DG44 cells. Glutamine synthetase (GS)-deficient derivatives of the mammalian cell, particularly of the CHO-DG44 and CHO-K1 cell are also encompassed. The mammalian cell may further comprise one or more expression cassette(s) encoding a heterologous protein, preferably a recombinant secreted therapeutic protein. The mammalian cells may also be murine cells such as murine myeloma cells, such as NS0 and Sp2/0 cells or the derivatives/progenies of any of such cell line. However, derivatives/progenies of those cells, other mammalian cells, including but not limited to human, mice, rat, monkey, and rodent cell lines, can also be used in the present invention, particularly for the production of biopharmaceutical proteins.

The term "growth phase" as used herein refers to the phase of cell culture where the cells proliferate exponentially and viable cell density in the bioreactor is increasing. Cells in culture usually proliferate following a standard growth pattern. The first phase of growth after the culture is seeded is the lag phase, which is a period of slow growth when the cells are adapting to the culture environment and preparing for fast growth. The lag phase is followed by the growth phase (also referred to as log phase or logarithmic phase), a period where the cells proliferate exponentially and consume the nutrients of the growth medium. In certain embodiments herein, the one or more additives of linoleic acid, arachidonic acid, and prostaglandin E2 may be added at the start of growth phase, or at some time during growth phase. In other embodiments, initial growth medium may comprise the one or more additives of linoleic acid, arachidonic acid, and prostaglandin E2 at the beginning of the growth phase, i.e., at the start of the cell culture.

The term "production phase" refers to the phase of cell culture which begins once the target cell density is reached and/or harvest is started. A typical target cell density is in the range of 10 x 10⁶ cells/ml to about 120 x 10⁶ cells/ml, but may be even higher. Thus, the target cell density according to the present invention is at least 10 x 10⁶ cells/ml, at least 20 x 10⁶ cells/ml, at least 30 x 10⁶ cells/ml, at least 40 x 10⁶ cells/ml or at least 50 x 10⁶ cells/ml. Most preferably the target cell density is about 30 to about 50 x 10⁶ cells/ml. Viable cell density is dependent on the perfusion rate and can be maintained at a constant level using regular or continuous cell bleeds. In certain embodiments herein, the one or more additives of linoleic acid, arachidonic acid, and prostaglandin E2 may be added at the start of production phase, or at some time during production phase.

The term "growth-arrest" as used herein refers to cells that are stopped from increasing in number, i.e., from cell division. The cell cycle comprises the interphase and the mitotic phase. The interphase consists of three phases: DNA replication is confined to S phase; G₁ is the gap between M phase and S phase, while G₂ is the gap between S phase and M phase. In M phase, the nucleus and then the cytoplasm divide. In the absence of a mitogenic signal to proliferate or in the presence of compounds that induce growth arrest the cell cycle arrests. The cells may partly disassemble their cell-cycle control system and exit from the cycle to a specialized, non-dividing state called G₀.

The term "bolus addition" as used herein refers to an addition that immediately adjusts the concentration in the cell culture to the desired concentration. According to the invention it means that the retinoid concentration in the cell culture is instantaneously adjusted to the retinoid concentration of the invention. This is to avoid a transitional phase wherein cells are cultured at a lower retinoid concentration that may result in unwanted proliferative activity.

In various aspects, the present invention may refer to "one or more of linoleic acid, arachidonic acid, and prostaglandin E2" in connection with the methods and cell culture media of the present disclosure. The phrase "one or more of" contemplates any of the following to be used in connection with the methods and media disclosed herein: linoleic acid alone; arachidonic acid alone; prostaglandin E2 alone; a double combination of linoleic acid and arachidonic acid; a double combination of linoleic acid and prostaglandin E2; a double combination of arachidonic acid and prostaglandin E2; and a triple combination of linoleic acid, arachidonic acid, and prostaglandin E2. The relative amounts, concentrations, and/or ratios of the additives when used in combination are described herein elsewhere.

Reference to "derivatives and/or precursors" of a compound, such as in reference to linoleic acid, arachidonic acid, or prostaglandin E2, refers to "derivatives" and "precursors". Derivatives are compounds which are derived from a starting compound by a chemical reaction and which are structural and/or functional analogs, i.e., having the same or approximately the same structure and function as the starting compound. Derivatives of linoleic acid, arachidonic acid, and prostaglandin E2 are compounds which are derived from any of these three compounds through a chemical reaction that changes or modifies the structure or atomic composition of the starting compound (e.g., addition or subtraction of a functional group) and which are structurally similar (i.e., structural analogs) and have the same or similar function (i.e., functional analog). Precursors are compounds that precede another (directly or indirectly) in a metabolic pathway. In the instant invention, linoleic acid is a precursor to arachidonic acid, which is a precursor to prostaglandin E2 ("PGE₂") on the same metabolic pathway - see Fig. 2. Referring to Fig. 2, additional precursors to prostaglandin E2 include PGG₂ (prostaglandin G₂) and PGH₂ (prostaglandin H₂), which are converted by COX-1 and COX-2 enzymes in the cell to form PGE₂. Thus, it is also described herein that metabolic precursors of each of linoleic acid, arachidonic acid and/or prostaglandin E2 may also be used in the herein described methods and media. The term "about" as used herein refers to a variation around the actual value provided and encompasses plus and minus 10% of the value.

### Cell culture methods

For the purposes of understanding, yet without limitation, it will be appreciated by the skilled practitioner that cell cultures and culturing runs for protein production can include at least three general types; namely, perfusion culture, batch culture and fed-batch culture.. In a perfusion culture, for example, fresh culture medium supplement is provided to the cells during the culturing period, while old culture medium is removed daily and the product is harvested, for example, daily or continuously. In perfusion culture, perfusion medium can be added daily and can be added continuously, i.e., as a drip or infusion. For perfusion culturing, the cells can remain in culture as long as is desired, so long as the cells remain alive and the environmental and culturing conditions are maintained. Since the cells grow continuously, it is typically required to remove cells during the run in order to maintain a constant viable cell density, which is referred to as cell bleed. The cell bleed contains product in the culture medium removed with the cells, which is typically discarded and hence wasted. Thus, maintaining the viable cell density during production phase without or with only minimal cell bleeding is advantageous and increases the total yield per run.

In batch culture, cells are initially cultured in medium and this medium is not removed, replaced, or supplemented, i.e., the cells are not "fed" with new medium, during or before the end of the culturing run. The desired product is harvested at the end of the culturing run.

For fed-batch cultures, the culturing run time is increased by supplementing the culture medium one or more times daily (or continuously) with fresh medium during the run, i.e., the cells are "fed" with new medium ("feeding medium") during the culturing period. Fed-batch cultures can include the various feeding regimens and times as described above, for example, daily, every other day, every two days, etc., more than once per day, or less than once per day, and so on. Further, fed-batch cultures can be fed continuously with feeding medium. The desired product is then harvested at the end of the culturing/production run.

According to the present invention, mammalian cells are cultured in perfusion culture. During heterologous protein production it is desirable to have a controlled system where cells are grown to a desired viable cell density and then the cells are switched to a growth-arrested, high productivity state where the cells use energy and substrates to produce the heterologous protein of interest rather than cell growth and cell division. Methods for accomplishing this goal, such as temperature shifts and amino acid starvation, are not always successful and can have undesirable effects on product quality. As described herein viable cell density during production phase can be maintained at a desirable level by performing a regular cell bleed. However, this results in discarding heterologous protein of interest. Cell growth-arrest during production phase results in a reduced need for a cell bleed and may even maintain cells in a more productive state.

In one aspect of the disclosure, a method of culturing mammalian cells expressing a heterologous protein in a perfusion cell culture is provided comprising culturing mammalian cells expressing a heterologous protein in a culture medium comprising an effective amount of one or more lipids or lipid metabolites wherein the one or more lipids or lipid metabolites comprises 500-2000 µM linoleic acid, 100-600 µM arachidonic acid and/or 0.0001-100 µM prostaglandin E2 and, wherein the lipid or lipid metabolite or combination thereof results in growth suppression and increased productivity, without the need for cell bleeding.

In another aspect of the disclosure, a method of culturing mammalian cells expressing a heterologous protein in a perfusion cell culture is provided comprising culturing mammalian cells expressing a heterologous protein in a perfusion culture medium comprising one or more of an effective amount of a lipid or lipid metabolite wherein the one or more lipids or lipid metabolites comprises 500-2000 µM linoleic acid, 100-600 µM arachidonic acid and/or 0.0001-100 µM prostaglandin E2.

In yet another aspect of the disclosure, a method of culturing mammalian cells expressing a heterologous protein in a perfusion cell culture is provided comprising culturing mammalian cells expressing a heterologous protein in a perfusion culture medium comprising an effective amount of 500-2000 µM linoleic acid.

In still another aspect of the disclosure, a method of culturing mammalian cells expressing a heterologous protein in a perfusion cell culture is provided comprising culturing mammalian cells expressing a heterologous protein in a perfusion culture medium comprising an effective amount of 100-600 µM arachidonic acid.

In another aspect of the disclosure, a method of culturing mammalian cells expressing a heterologous protein in a perfusion cell culture is provided comprising culturing mammalian cells expressing a heterologous protein in a perfusion culture medium comprising an effective amount of 0.0001-100 µM prostaglandin E2.

According to another aspect of the disclosure, a method of enhancing the specific productivity of a perfusion cell culture is provided comprising (a) culturing recombinant mammalian cells encoding a protein of interest in a perfusion growth medium until a desired cell density is reached, and (b) introducing an effective amount of one or more of a lipid or lipid metabolite selected from the group consisting of: linoleic acid, arachidonic acid, and prostaglandin E2 into the growth medium, wherein the one or more lipids or lipid metabolites comprises 500-2000 µM linoleic acid, 100-600 µM arachidonic acid and/or 0.0001-100 µM prostaglandin E2. In some embodiments, the added lipids and/or lipid metabolites result in the suppression of cell growth such that a steady state is reached, thereby achieving enhanced specific productivity of the perfusion cell culture.

According to yet another aspect of the disclosure, a perfusion cell culture medium for culturing mammalian cells and producing therapeutic proteins with improved productivity is provided. The cell culture medium comprises one or more of an effective amount of one or more lipids or lipid metabolites comprising 500-2000 µM linoleic acid, 100-600 µM arachidonic acid and/or 0.0001-100 µM prostaglandin E2, wherein the perfusion cell culture medium is protein-free but optionally includes insulin and/or insulin-like growth factor. In some embodiments, the added lipids or lipid metabolites or combinations thereof result in growth suppression and increased productivity. In some embodiments, the growth suppression may result in reducing or eliminating the need for cell bleeding particularly with regard to perfusion cell cultures. The cell culture medium can be used in continuous cultures, including continuous perfusion cultures.

The term "cell culture" is not particularly limited and may encompass all forms and techniques of cell culture, including but not limited to, fed-batch, batch, perfusion, continuous, finite, suspension, adherent or monolayer, anchorage-dependent, and 3D cultures. The present invention relates to perfusion cell culture. In a preferred embodiment, the cell culture is a continuous perfusion cell culture.

In various embodiments, the effective amount of linoleic acid is from about 1800-2000 µM. In one embodiment, the linoleic acid is in a concentration of 500-2000 µM.

In other embodiments, the effective amount of arachidonic acid is from about 150-500 µM. In one embodiment, the arachidonic acid is in a concentration of 100-600 µM.

In other embodiments, the effective amount of prostaglandin E2 is from about 0.001 µM to 60 µM. In still other embodiments, the effective amount of prostaglandin E2 is 0.0001 µM or more, 0.001 µM or more, 0.01 µM or more, 0.1 µM or more, 1 µM or more, 10 µM or more, 20 µM or more, 30 µM or more, 40 µM or more, 50 µM or more, 60 µM or more, 70 µM or more, 80 µM or more, 90 µM or more, wherein the upper limit of each range is 100 µM or less. In yet other embodiments, the effective amount of prostaglandin E2 is from about 1-100 µM, or from about 1-50 µM, or about 1-25 µM, or from about 0.0001-0.001 µM, to about 0.0005-0.01 µM, to about 0.005-0.1 µM, to about 0.05-1.0 µM to about 0.5-100 µM. In one embodiment, the prostaglandin E2 is in a concentration of 0.0001-100 µM.

In some embodiments, the culture medium comprises at least two of the lipids or lipid metabolites. In one embodiment, the culture medium comprises arachidonic acid at a concentration of 100-300 µM and linoleic acid at a concentration of 500-1800 µM. In another embodiment, the culture medium comprises prostaglandin E2 at a concentration of 0.0001-0.0009 µM in combination with either linoleic acid at a concentration of 500-1800 µM or arachidonic acid at a concentration of 100-150 µM.

In still another embodiment, the culture medium comprises all three lipids or lipid metabolites. In one embodiment, the culture medium comprises linoleic acid in a concentration of 500-2000 µM, the arachidonic acid at a concentration of 100-300 µM, and the prostaglandin E2 at a concentration of 0.0001-0.0009 µM.

In another aspect of the disclosure, a method of culturing mammalian cells expressing a heterologous protein in a perfusion cell culture is provided comprising culturing mammalian cells expressing a heterologous protein in a culture medium comprising an effective amount one or more of linoleic acid in a concentration of 500-2000 µM, arachidonic acid in a concentration of 100-300 µM, and prostaglandin E2 in a concentration of 0.0001-0.0009 µM, wherein the lipid or lipid metabolite or combination thereof results in growth suppression and increased productivity, without the need for cell bleeding.

In other embodiments, the present invention embraces the following amounts of linoleic acid, arachidonic acid, and/or prostaglandin E2 expressed in terms of molar ratios or weight ratios. These ratios may be used to combine at least two of linoleic acid, arachidonic acid, or prostaglandin E2, or all three additives in the cell culture media. One of ordinary skill in the art may add these components in combinations of two or three additives in the following ratios determined as a ratio of the total weight added, or based on their molar ratios. The components may be added at the same time or at different times. The following ratios of linoleic acid to arachidonic acid to prostaglandin E2 (linoleic acid : arachidonic acid : prostaglandin E2) may be used: (1) 1:1:1; (2) 5-1:1:1; (3) 10-1:1:1; (4) 100-1:1:1; (5) 1000-1:1:1; (6) 1:5-1:1; (7) 1:10-1:1; (8) 1:100-1:1; (9) 1:1000-1:1; (10) 1:1:0.1-0.01; (11) 1:1:0.1-0.001; (12) 1:1:0.1-0.0001; (13) 1:1:0.1-0.00001; (14) 1:1:0.1-0.0000001; (15) 1-1000:1-1000:1-0.1; (16) 1-1000:1-1000:0.1-0.01; (17) 1-1000:1-1000:0.1-0.001; (18) 1-1000:1-1000:0.1-0.0001; (19) 1-1000:1-1000:0.1-0.00001; (20) 1-1000:1-1000:0.1-0.0000001; (16) 0.1-1000:1-1000:1-0.1; (17) 0.01-1000:1-1000:0.1-0.01; (18) 0.001-1000:1-1000:0.1-0.001; (19) 1-1000:0.1-1000:0.1-0.0001; (20) 1-1000:0.01-1000:0.1-0.00001; (21) 1-1000:0.001-1000:0.1-0.0000001; (22) 1:1:0.1-0.0000001; (23) 5-1:1:0.1-0.0000001; (24) 10-1:1:0.1-0.0000001; (25) 100-1:1:0.1-0.0000001; (26) 1000-1:1:0.1-0.0000001; (27) 1:5-1:0.1-0.0000001; (28) 1:10-1:0.1-0.0000001; (29) 1:100-1:0.1-0.0000001; (30) 1:1000-1:0.1-0.0000001. In certain embodiments, the invention may utilize a specific ratio falling in the range of ratios of: 5:1:0.000001 through 2:0.6:0.1, which are ratios which correspond to using a concentration of linoleic acid at 500-2000 µM, a concentration of arachidonic acid at 100-600 µM, and prostaglandin E2 at 0.0001-100 µM. Other ratios are contemplated and the above listing of ratios is not intended to limit the invention in any way.

In other embodiments, the disclosure provides a method of culturing mammalian cells expressing a heterologous protein in a perfusion cell culture comprising: (a) culturing mammalian cells expressing a heterologous protein in a serum-free culture medium; (b) culturing the mammalian cells during growth phase by perfusion with a serum-free perfusion medium; and (c) maintaining the mammalian cells during production phase by perfusion with a serum-free perfusion medium comprising one or more of linoleic acid in a concentration of 500-2000 µM, arachidonic acid in a concentration of 100-300 µM, and prostaglandin E2 in a concentration of 0.0001-0.0009 µM, wherein step (b) is optional. In one embodiment of the method prior to step (c) the lipid or lipid metabolites are added by bolus.

Also provided herein is a method of reducing cell bleeding in a perfusion cell culture and/or increasing protein production in a perfusion cell culture expressing a heterologous protein comprising: (a) culturing mammalian cells expressing a heterologous protein in a serum-free culture medium; (b) culturing the mammalian cells during growth phase by perfusion with a serum-free perfusion medium; and (c) maintaining the mammalian cells during production phase by perfusion with a serum-free perfusion medium one or more of linoleic acid in a concentration of 500-2000 µM, arachidonic acid in a concentration of 100-300 µM, and prostaglandin E2 in a concentration of 0.0001-0.0009 µM, wherein step (b) is optional. In one embodiment of the method prior to step (c) the lipid or lipid metabolites are added by bolus.

It is also encompassed by the invention that the perfusion culture is inoculated with a very high cell density and perfusion is started immediately or shortly after inoculation of mammalian cells expressing a heterologous protein in a serum-free culture medium. Further, step (b) culturing the mammalian cells during growth phase by perfusion with a serum-free perfusion medium may be optional so that the mammalian cells are immediately cultured according to step (c) during production phase by perfusion with a serum-free perfusion medium comprising one or more of linoleic acid in a concentration of 500-2000 µM, arachidonic acid in a concentration of 100-300 µM, and prostaglandin E2 in a concentration of 0.0001-0.0009 µM. Preferably, prior to step (c) the concentration of the lipids in the mammalian cell culture is adjusted to the desired concentration preferably by bolus addition.

Thus, also provided herein is a method of culturing mammalian cells expressing a heterologous protein and/or reducing cell bleeding in a perfusion cell culture and/or increasing protein production in a perfusion cell culture expressing a heterologous protein comprising: (a) inoculating mammalian cells expressing a heterologous protein in a serum-free culture medium; (b) optionally culturing the mammalian cells during growth phase by perfusion with a serum-free perfusion medium; and (c) maintaining the mammalian cells during production phase by perfusion with a serum-free perfusion medium comprising one or more of linoleic acid in a concentration of 500-2000 µM, arachidonic acid in a concentration of 100-300 µM, and prostaglandin E2 in a concentration of 0.0001-0.0009 µM. Preferably, prior to step (c) the concentration of the lipids in the mammalian cell culture is adjusted to the desired concentration preferably by bolus addition. Increasing the protein production encompasses an increased protein product yield over the perfusion run or over a certain period of time. It also encompasses an increased specific protein production per cell.

According to the methods of the invention, culturing the mammalian cells in step (a) may be limited to inoculating mammalian cells expressing a heterologous protein in a serum-free medium and hence does not need to include an actual culturing step prior to the start of perfusion. Further according to the methods of the invention, maintaining the mammalian cells during production phase by perfusion includes culturing the mammalian cells during production phase by perfusion at a constant viable cell density.

The production phase starts once the target cell density is reached. Preferably step (c) is started once the target cell density is reached. It may be started at a cell density of 10 x 10⁶ cells/ml to about 120 x 10⁶ cells/ml or even higher. Preferably step (c) is initiated at a cell density of at least 10 x 10⁶ cells/ml, at least 20 x 10⁶ cells/ml, at least 30 x 10⁶ cells/ml, at least 40 x 10⁶ cells/ml or at least 50 x 10⁶ cells/ml. Most preferably step (c) is initiated at a cell density of about 30 to about 50 x 10⁶ cells/ml.

As already explained above, the methods of the invention may further comprise that in step (c) the cell density is maintained by cell bleeding. The cell density referred to in this context is the viable cell density, which may be determined by any method known in the art. For example the calculation governing the cell bleed rate may be based on maintaining the INCYTE^{™} viable cell density probe (HAMILTON^{®} COMPANY) or FUTURA^{™} biomass capacitance probe value (ABER^{®} instruments) which corresponded to the target VCD, or a daily cell and viability count can be taken off-line via any cell counting device, such as haemocytometer, VI-CELL XR^{™} (BECKMAN COULTER^{®}), CEDEX HI-RES^{™} (ROCHE^{®}), or VIACOUNT^{™} assay (EMD MILLIPORE^{®} GUAVA EASYCYTE^{®}). Using the methods of the present invention the cell bleeding is reduced compared to a control perfusion cell culture, wherein a control perfusion cell culture is a perfusion cell culture that is cultured under the same conditions using the same serum-free perfusion medium without the added lipids according to the invention. More specifically the cell bleeding is reduced compared to a control perfusion cell culture, wherein a control perfusion cell culture is a perfusion cell culture that is cultured under the same conditions using the same serum-free perfusion medium with the added lipids at the desired concentrations.

For the same reason and to allow growth during growth phase, in certain embodiments, the lipid concentrations of the serum-free perfusion medium of the invention should be avoided in the inoculation medium and during growth phase. Thus, the serum-free culture medium of step (a) and the serum-free perfusion medium of step (b) should comprise no lipids (i.e., no linoleic acid, arachidonic acid, or prostaglandin E2). Preferably the serum-free culture medium of step (a) and the serum-free perfusion medium of step (b) comprise no lipids (i.e., no linoleic acid, arachidonic acid, or prostaglandin E2).

The osmolarity of the serum-free perfusion medium of the invention should be in the range of between 300 and 1400 mOsmol/kg, preferably between 300 and 500 mOsmol/kg, more preferably between 330 and 450 mOsmol/kg and even more preferably between 360 and 390 mOsmol/kg. Wherein the osmolarity is provided as mOsmol/kg water.

A perfusion culture typically starts with an inoculation culture as batch culture. Perfusion may start immediately or after one or more days. Typically, perfusion starts on or after day 2 of the cell culture. In one embodiment the perfusion in step (b) begins on or after day 2 of the cell culture. Once the target cell density is reached, growth arrest is induced by increasing the concentration of one or more of linoleic acid, arachidonic acid, or prostaglandin E2 in the cell culture medium to a desired concentration. Preferably this increase in lipid/lipid metabolite concentration to the desired concentration is instantaneously, such as by bolus addition. This adjustment in the concentration of linoleic acid, arachidonic acid, and/or prostaglandin E2 in the cell culture can occur in one embodiment once a certain desired concentration of cells is reached, i.e., end the end of the growth phase. During the next phase, i.e., the production phase, the mammalian cells can be cultured by perfusion with a serum-free perfusion medium comprising one or more of the exogenously added linoleic acid, arachidonic acid, or prostaglandin E2 at the desired concentration. The lipids/lipid metabolites may also be added to the culture from the start and hence may be added to the serum-free culture medium used for inoculation or to the serum-free perfusion medium before growth arrest is induced by bolus addition as described above and hence before the target cell density is reached. At the latest, the lipids/lipid metabolites must be added with the serum-free perfusion medium once the target cell density is reached. In another embodiment, the desired lipid/lipid metabolite concentration in the mammalian cell culture is reached before the target cell density is reached and before growth arrest is induced by increasing the lipids/lipid metabolites in the mammalian cell culture.

Typically, the mammalian cell culture according to the invention comprises continuous perfusion of the cell culture. The perfusion rate may increase after perfusion has started. Typically, a higher perfusion rate supports a higher viable cell density and therefore allows for a higher target cell density. The perfusion rate may increase from less or equal to 0.5 vessel volumes per day to 5 vessel volumes per day. Preferably the perfusion rate increases from less than or equal to 0.5 vessel volumes per day to 2 vessel volumes per day.

### Bioreactors

According to the methods of the present invention, any cell perfusion bioreactor and cell retention device may be used for perfusion culture. The bioreactors used for perfusion are not very different from those used for batch/fed-batch cultures, except that they are more compact in size and are connected to a cell retention device. The methods for retaining cells inside the bioreactor are primarily determined by whether the cells are growing attached to surfaces or growing in either single cell suspension or cell aggregates. While most mammalian cells historically were grown attached to a surface or a matrix (heterogenous cultures), efforts have been made to adapt many industrial mammalian cell lines to grow in suspension (homogenous cultures), mainly because suspension cultures are easier to scale-up. Thus, the cells used in the methods of the invention are preferably grown in suspension. Without being limited thereto, exemplary retention systems for cells grown in suspension are spin filter, external filtration such as tangential flow filtration (TFF), alternating tangential flow (ATF) system, cell sedimentation (vertical sedimentation and inclined sedimentation), centrifugation, ultrasonic separation and hydrocyclones. Perfusion systems can be categorized into two categories, filtration based systems, such as spin filters, external filtration and ATF, and open perfusion systems, such as gravitational settlers, centrifuges, ultrasonic separation devices and hydroclones. Filtration-based systems show a high degree of cell retention and it does not change with the flow rate. However, the filters may clog and hence the cultivation run is limited in length or the filters need to be exchanged. An example for an ATF system is the XCELL^{™} ATF system from REPLIGEN^{™} and an example for a TFF system is the TFF system from LEVITRONIX^{®} using a centrifugal pump. A cross-flow filter, such as a hollow fiber (HF) or a flat plate filter may be used with ATF and TFF systems. Specifically a hollow fiber, made of modified polyethersulfone (mPES), polyethersulfone (PES), or polysulfone (PE), can be used with ATF and TFF systems. Pore sizes of the HF can range from several hundred kDa to 15 µM. Open perfusion systems do not clog and hence could at least theoretically be operated indefinitely. However, the degree of cell retention is reduced at higher perfusion rates. Currently there are three systems that can be used at industrial scale, alternating tangential filters (ATF), gravitational (particularly inclined settlers) and centrifuges. Cell retention devices suitable for heterogenous or homogenous cultures are described in more detail by Kompala and Ozturk (Cell Culture Technology for Pharmaceutical and Cell-Based Therapies, (2006), Taylor & Francis Group, LLC, pages 387-416). The perfusion culture is not a true steady state process, with the total and viable cell concentration reaching a steady state only when a cell bleed stream is removed from the bioreactor.

Physical parameters such as pH, dissolved oxygen and temperature in a perfusion bioreactor should be monitored on-line and controlled in real time. Determination of cell density, viability, metabolite, and product concentrations may be performed using off-line or on-line sampling. When the perfusion operation starts with continuous harvest and feeding the perfusion rate typically refers to the harvest flow rate, which may be manually set to a desired value. For example, a weight control for the bioreactor may activate the feed pump so that a constant volume in the bioreactor can be maintained. Alternatively, a level control can be achieved by pumping out culture volume above a predetermined level. The perfusion rate in the bioreactor must be adjusted to deliver sufficient nutrients to the cells. As the cell density increases in the bioreactor, the perfusion rate must be increased.

Perfusion rate may be controlled, e.g., using cell density measurements, pH measurements, oxygen consumption or metabolite measurements. Cell density is the most important measurement used for perfusion rate adjustments. Depending on how the cell density measurements are conducted, perfusion rates can be adjusted daily or in real time. Several on-line probes have been developed for the estimation of cell density and are known to the person skilled in the art, such as a capacitance probe, e.g., an INCYTE^{™} viable cell density probe (HAMILTON^{®} COMPANY) or FUTURA^{™} biomass capacitance probe value (ABER^{®} instruments). These cell density probes can also be used to control the cell density at a desired set point by removing excess cells from the bioreactor, i.e., the cell bleed. Thus, the cell bleed is determined by the specific growth rate of the mammalian cells in culture. The cell bleed is typically not harvested and therefore considered as waste.

The methods of the present invention further comprise harvesting the heterologous protein from the perfusion cell culture. The invention contemplates any suitable method for harvesting and purifying the protein of interest. The harvesting may also occur intermittently throughout the cell culture life cycle, or at the end of the cell culture. Harvesting is preferably done continuously from the permeate, which is the supernatant produced after cells have been recovered by a cell retention device. Due to the lower product residence time of the product proteins in the cell culture inside the perfusion bioreactor compared to fed-batch, the exposure to proteases, sialidases and other degrading proteins is minimized, which may result in better product quality of heterologous proteins produced in perfusion culture.

### Cell culture medium

The present invention contemplates the use of any suitable cell culture medium, including commercially-available medium and the like. Any of the medium contemplated herein should be capable of being modified by the addition of the lipids and/or lipid metabolites of the invention (e.g., linoleic acid, arachidonic acid, and prostaglandin E2, at the desired and effective concentrations defined herein). The type of base cell culture medium used may depend upon the type or format of cell culture used, e.g., batch, fed-batch, or perfusion. The skilled person will be able to select a suitable base medium.

In one embodiment, a serum-free perfusion medium can be used. The serum-free perfusion medium used in the methods of the invention may be chemically defined and/or hydrolysate-free. Hydrolysate-free means that the medium does not contain protein hydrolysates from animal, plant (soybean, potato, rice), yeast or other sources. Typically, a chemically defined medium is hydrolysate-free. In any case the serum-free perfusion medium should be free of compounds derived from animal sources, particularly proteins or peptides derived and isolated from an animal (this does not include recombinant proteins produced by the cell culture). Preferably the serum-free perfusion medium is protein-free or protein-free except for recombinant insulin and/or insulin-like growth factor. More preferably the serum-free perfusion medium is chemically defined and protein-free or protein-free except for recombinant insulin and/or insulin-like growth factor. This also applies to the serum-free culture medium of step (a) and the serum-free perfusion medium of step (b).

In yet another aspect, the disclosure provides a use of the serum-free perfusion medium of the invention for culturing mammalian cells in a perfusion culture during production phase or for reducing the total cell bleed volume in a perfusion culture. Alternatively, the disclosure provides a use of the serum-free perfusion medium of the invention for increasing protein production in a perfusion cell culture.

In various embodiments, the effective amount of linoleic acid in the medium when added is from about 1800-2000 µM (per final concentration in the medium). In one embodiment, the linoleic acid is at a concentration of 500-2000 µM.

In other embodiments, the effective amount of arachidonic acid is from about 150-500 µM. In one embodiment, the arachidonic acid is at a concentration of 100-600 µM.

In other embodiments, the effective amount of prostaglandin E2 is from about 0.001 µM to 60 µM. In still other embodiments, the effective amount of prostaglandin E2 is 0.001 µM, 10 µM, 20 µM, or 60 µM. In yet other embodiments, the effective amount of prostaglandin E2 is from about 1-100 µM, or from about 1-50 µM, or about 1-25 µM, or from about 0.0001-0.001 µM, to about 0.0005-0.01 µM, to about 0.005-0.1 µM, to about 0.05-1.0 µM, to about 0.5-100 µM. In one embodiment, the prostaglandin E2 is at a concentration of 0.0001-100 µM.

In some embodiments, the culture medium comprises at least two of the lipids or lipid metabolites, in combinations of any of the above concentrations or concentration ranges. In one embodiment, the culture medium comprises arachidonic acid at a concentration of 100-300 µM and linoleic acid at a concentration of 500-1800 µM. In another embodiment, the culture medium comprises prostaglandin E2 at a concentration of 0.0001-0.0009 µM in combination with either linoleic acid at a concentration of 500-1800 µM or arachidonic acid at a concentration of 100-150 µM.

In still another embodiment, the culture medium comprises all three lipids or lipid metabolites, in combinations of any of the above concentrations or concentration ranges. In one embodiment, the culture medium comprises linoleic acid in a concentration of 500-2000 µM, the arachidonic acid at a concentration of 100-300 µM, and the prostaglandin E2 at a concentration of 0.0001-0.0009 µM.

In certain embodiments, a cell culture medium or a method of the invention may utilize a cell culture medium that includes a decrease in the sodium-to-potassium ratio to decrease cell growth and improve cell specific productivity (i.e., increased potassium concentration; a.k.a. "K-pop" media as recited in FIG. 5). For example, the medium described in U.S. provisional application No. 62/479,422, filed March 31, 2017, may be utilized by incorporating the lipids and/or lipid metabolites described herein. The methods and lipid additives described herein may be used to further reduce viable cell density, cell growth, and improve cell specific productivity.

In other embodiments, a cell culture medium or a method according to the invention may utilize vitamin additives (e.g., Vitamin A or retinyl acetate) to slow cell growth in perfusion cell culture. The methods and lipid additives described herein may be used to further reduce viable cell density, cell growth, and improve cell specific productivity in such media. An example of such a medium can be found in U.S. provisional application No 62/479,414, filed March 31, 2017.

The cell culture methods described herein contemplate any suitable manner and timing by which to add, mix, combine, or otherwise obtain a particular cell medium comprising the lipids and/or lipid metabolites of the invention (e.g., linoleic acid, arachidonic acid, and/or prostaglandin E2). For example, the lipids and/or lipid metabolites of the invention may be mixed into the initial culture medium at their desired and/or effective concentrations prior to or at the start of commencing the cell culture. In another embodiment, the lipids and/or lipid metabolites of the invention may be added or otherwise mixed or combined into an actively growing cell culture at some point downstream of cell culture inoculation. In still another embodiment, it is envisioned that once added, the optimal effective concentration of the lipids and/or lipid metabolites of the invention may be maintained at the effective desired concentrations by continuing to add additional increments of the lipids and/or lipid metabolites to the cell culture medium. In various embodiments, the lipids and/or lipid metabolites can be prepared in liquid medium (which can be the same of the cell culture medium, or different, and/or may contain appropriate effective amounts of lipid solvents, such as ethanol, acetone, benzene, or other lipid-dissolving solvents) at a first, higher concentration, which can be referred to as a "stock concentration" or a "stock material." To adjust the cell culture medium to the desired "working" concentration of the lipids and/or lipid metabolites, one of ordinary skill in the art would then transfer an appropriate volume or quantity of the stock material to the cell culture medium to achieve the desired working concentration. In some cases, it may be desirable to add the stock material at a rate which avoids introducing too high a local concentration of the lipids and/or lipid metabolites (or the lipid-dissolving solvent used in the stock medium) which could possibly be detrimental to cells that may come into contact with the temporary local high concentration of the stock material being added to the cell culture. In other embodiments, the invention contemplates simply adding a bolus addition of a stock material to the cell culture.

In addition, regarding the configuration of the cell culture bioreactors with regard to adding the lipids and/or lipid metabolites of the invention, e.g., as a stock concentration material, the lipids and/or lipid metabolites may be added through the same valve or entry point as the cell culture media. In other embodiments, the lipids and/or lipid metabolites may be added through an independent valve or entry point. The cell culture bioreactors may be configured to accommodate the step of adding the lipids and/or lipid metabolites at any time of the cell culture cycle, including at the start of the cell culture, during the growth phase of the cell culture, and during the production phase of the cell culture. The bioreactors may also be configured for a single bolus addition of lipids and/or lipid metabolites through a shared valve or port, or through a separate or independent valve or port. The bioreactors may also be configured to accommodate an intermittent series of additions or a continuous stream of additions of lipids and/or lipid metabolites to cell culture medium through a shared valve or port, or through a separate or independent valve of port. The input of stock concentration of lipids and/or lipid metabolites of the invention may be configured to be added manually, automatically, or semi-automatically by any known means.

### Expression products

The heterologous protein produced by the methods and uses of the present invention may be any secreted protein, preferably it is a therapeutic protein. Since most therapeutic proteins are recombinant therapeutic proteins, it is most preferably a recombinant therapeutic protein. Examples for therapeutic proteins are without being limited thereto antibodies, fusion proteins, cytokines and growth factor.

The therapeutic protein produced in the mammalian cells according to the methods of the invention includes, but is not limited to an antibodies or a fusion protein, such as a Fc-fusion proteins. Other secreted recombinant therapeutic proteins can be for example enzymes, cytokines, lymphokines, adhesion molecules, receptors and derivatives or fragments thereof, and any other polypeptides and scaffolds that can serve as agonists or antagonists and/or have therapeutic or diagnostic use.

Other recombinant proteins of interest are for example, without being limited thereto: insulin, insulin-like growth factor, hGH, tPA, cytokines, such as interleukins (IL), e.g. IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosis factor (TNF), such as TNF alpha and TNF beta, TNF gamma, TRAIL; G-CSF, GM-CSF, M-CSF, MCP-1, and VEGF. Also included is the production of erythropoietin or any other hormone growth factors and any other polypeptides that can serve as agonists or antagonists and/or have therapeutic or diagnostic use.

A preferred therapeutic protein is an antibody or a fragment or derivative thereof, more preferably an IgG1 antibody. Thus, the invention can be advantageously used for production of antibodies such as monoclonal antibodies, multi-specific antibodies, or fragments thereof, preferably of monoclonal antibodies, bi-specific antibodies or fragments thereof. Exemplary antibodies within the scope of the present invention include but are not limited to anti-CD2, anti-CD3, anti-CD20, anti-CD22, anti-CD30, anti-CD33, anti-CD37, anti-CD40, anti-CD44, anti-CD44v6, anti-CD49d, anti-CD52, anti-EGFR1 (HER1), anti-EGFR2 (HER2), anti-GD3, anti-IGF, anti-VEGF, anti-TNFalpha, anti-IL2, anti-IL-5R or anti-IgE antibodies, and are preferably selected from the group consisting of anti-CD20, anti-CD33, anti-CD37, anti-CD40, anti-CD44, anti-CD52, anti-HER2/neu (erbB2), anti-EGFR, anti-IGF, anti-VEGF, anti-TNFalpha, anti-IL2 and anti-IgE antibodies.

Antibody fragments include e.g. "Fab fragments" (Fragment antigen-binding = Fab). Fab fragments consist of the variable regions of both chains, which are held together by the adjacent constant region. These may be formed by protease digestion, e.g. with papain, from conventional antibodies, but similarly Fab fragments may also be produced by genetic engineering. Further antibody fragments include F(ab')2 fragments, which may be prepared by proteolytic cleavage with pepsin.

Using genetic engineering methods it is possible to produce shortened antibody fragments which consist only of the variable regions of the heavy (VH) and of the light chain (VL). These are referred to as Fv fragments (Fragment variable = fragment of the variable part). Since these Fv-fragments lack the covalent bonding of the two chains by the cysteines of the constant chains, the Fv fragments are often stabilized. It is advantageous to link the variable regions of the heavy and of the light chain by a short peptide fragment, e.g. of 10 to 30 amino acids, preferably 15 amino acids. In this way a single peptide strand is obtained consisting of VH and VL, linked by a peptide linker. An antibody protein of this kind is known as a single-chain-Fv (scFv). Examples of scFv-antibody proteins are known to the person skilled in the art.

Preferred therapeutic antibodies according to the invention are bispecific antibodies. Bispecific antibodies typically combine antigen-binding specificities for target cells (e.g., malignant B cells) and effector cells (e.g., T cells, NK cells or macrophages) in one molecule. Exemplary bispecific antibodies, without being limited thereto are diabodies, BiTE (Bi-specific T-cell Engager) formats and DART (Dual-Affinity Re-Targeting) formats. The diabody format separates cognate variable domains of heavy and light chains of the two antigen binding specificities on two separate polypeptide chains, with the two polypeptide chains being associated non-covalently. The DART format is based on the diabody format, but it provides additional stabilization through a C-terminal disulfide bridge.

Another preferred therapeutic protein is a fusion protein, such as an Fc-fusion protein. Thus, the invention can be advantageously used for production of fusion proteins, such as Fc-fusion proteins. Furthermore, the method of increasing protein producing according to the invention can be advantageously used for production of fusion proteins, such as Fc-fusion proteins.

The effector part of the fusion protein can be the complete sequence or any part of the sequence of a natural or modified heterologous protein or a composition of complete sequences or any part of the sequence of a natural or modified heterologous protein. The immunoglobulin constant domain sequences may be obtained from any immunoglobulin subtypes, such as IgG1, IgG2, IgG3, IgG4, lgA1 or IgA2 subtypes or classes such as IgA, IgE, IgD or IgM. Preferentially they are derived from human immunoglobulin, more preferred from human IgG and even more preferred from human IgG1 and IgG2 Non-limiting examples of Fc-fusion proteins are MCP1-Fc, ICAM-Fc, EPO-Fc and scFv fragments or the like coupled to the CH2 domain of the heavy chain immunoglobulin constant region comprising the N-linked glycosylation site. Fc-fusion proteins can be constructed by genetic engineering approaches by introducing the CH2 domain of the heavy chain immunoglobulin constant region comprising the N-linked glycosylation site into another expression construct comprising for example other immunoglobulin domains, enzymatically active protein portions, or effector domains. Thus, an Fc-fusion protein according to the present invention comprises also a single chain Fv fragment linked to the CH2 domain of the heavy chain immunoglobulin constant region comprising e.g. the N-linked glycosylation site.

### Recovery of and formulation of expression products

In a further aspect a method of producing a therapeutic protein is provided using the methods of the invention and optionally further comprising a step of purifying and formulating the therapeutic protein into a pharmaceutically acceptable formulation.

The therapeutic protein, especially the antibody, antibody fragment or Fc-fusion protein is preferably recovered/isolated from the culture medium as a secreted polypeptide. It is necessary to purify the therapeutic protein from other recombinant proteins and host cell proteins to obtain substantially homogenous preparations of the therapeutic protein. As a first step, cells and/or particulate cell debris are removed from the culture medium. Further, the therapeutic protein is purified from contaminant soluble proteins, polypeptides and nucleic acids, for example, by fractionation on immunoaffinity or ion-exchange columns, ethanol precipitation, reverse phase HPLC, Sephadex chromatography, and chromatography on silica or on a cation exchange resin such as DEAE. Methods for purifying a heterologous protein expressed by mammalian cells are known in the art.

### Expression vectors

In one embodiment the heterologous protein expressed using the methods of the invention is encoded by one or more expression cassette(s) comprising a heterologous polynucleotide coding for the heterologous protein. The heterologous protein may be placed under the control of an amplifiable genetic selection marker, such as dihydrofolate reductase (DHFR), glutamine synthetase (GS). The amplifiable selection marker gene can be on the same expression vector as the heterologous protein expression cassette. Alternatively, the amplifiable selection marker gene and the heterologous protein expression cassette can be on different expression vectors, but integrate in close proximity into the host cell's genome. Two or more vectors that are co-transfected simultaneously, for example, often integrate in close proximity into the host cell's genome. Amplification of the genetic region containing the secreted therapeutic protein expression cassette is then mediated by adding the amplification agent (e.g., MTX for DHFR or MSX for GS) into the cultivation medium.

Sufficiently high stable levels of a heterologous protein expressed by a mammalian cell may also be achieved, e.g., by cloning multiple copies of the heterologous protein encoding-polynucleotide into an expression vector. Cloning multiple copies of the heterologous protein-encoding polynucleotide into an expression vector and amplifying the heterologous protein expression cassette as described above may further be combined.

### Mammalian cell lines

Mammalian cells as used herein are mammalian cells lines suitable for the production of a secreted recombinant therapeutic protein and may hence also be referred to as "host cells". Preferred mammalian cells according to the invention are rodent cells such as hamster cells. The mammalian cells are isolated cells or cell lines. The mammalian cells are preferably transformed and/or immortalized cell lines. They are adapted to serial passages in cell culture and do not include primary non-transformed cells or cells that are part of an organ structure. Preferred mammalian cells are BHK21, BHK TK-, CHO, CHO-K1, CHO-DXB11 (also referred to as CHO-DUKX or DuxB11), a CHO-S cell and CHO-DG44 cells or the derivatives/progenies of any of such cell line. Particularly preferred are CHO cells, such as CHO-DG44, CHO-K1 and BHK21, and even more preferred are CHO-DG44 and CHO-K1 cells. Most preferred are CHO-DG44 cells. Glutamine synthetase (GS)-deficient derivatives of the mammalian cell, particularly of the CHO-DG44 and CHO-K1 cell are also encompassed. In one embodiment of the invention the mammalian cell is a Chinese hamster ovary (CHO) cell, preferably a CHO-DG44 cell, a CHO-K1 cell, a CHO DXB11 cell, a CHO-S cell, a CHO GS deficient cell or a derivative thereof.

The mammalian cell may further comprise one or more expression cassette(s) encoding a heterologous protein, such as a therapeutic protein, preferably a recombinant secreted therapeutic protein. The host cells may also be murine cells such as murine myeloma cells, such as NS0 and Sp2/0 cells or the derivatives/progenies of any of such cell line. Non-limiting examples of mammalian cells which can be used in the meaning of this invention are also summarized in Table 1. However, derivatives/progenies of those cells, other mammalian cells, including but not limited to human, mice, rat, monkey, and rodent cell lines, can also be used in the present invention, particularly for the production of biopharmaceutical proteins.

**Table 1: Mammalian production cell lines**

| Cell line | Order Number |
|---|---|
| NS0 | ECACC No. 85110503 |
| Sp2/0-Ag14 | ATCC CRL-1581 |
| BHK21 | ATCC CCL-10 |
| BHK TK⁻ | ECACC No. 85011423 |
| HaK | ATCC CCL-15 |
| 2254-62.2 (BHK-21 derivative) | ATCC CRL-8544 |
| CHO | ECACC No. 8505302 |
| CHO wild type | ECACC 00102307 |
| CHO-K1 | ATCC CCL-61 |
| CHO-DUKX (= CHO duk⁻, CHO/dhfr^{-,},CHO-DXB11) | ATCC CRL-9096 |
| CHO-DUKX 5A-HS-MYC | ATCC CRL-9010 |
| CHO-DG44 | Urlaub G, et al., 1983. Cell. 33:405-412. |
| CHO Pro-5 | ATCC CRL-1781 |
| CHO-S | Life Technologies A1136401; CHO-S is derived from CHO variant Tobey et al. 1962 |
| V79 | ATCC CCC-93 |
| B14AF28-G3 | ATCC CCL-14 |
| HEK 293 | ATCC CRL-1573 |
| COS-7 | ATCC CRL-1651 |
| U266 | ATCC TIB-196 |
| HuNS1 | ATCC CRL-8644 |
| CHL | ECACC No. 87111906 |
| CAP¹ | Wölfel J, et al., 2011. BMC Proc. 5(Suppl 8):P133. |
| PER.C6^{®} | Pau et al., 2001. Vaccines. 19: 2716-2721. |
| H4-II-E | ATCC CRL-1548 |
| | ECACC No.87031301 |
| | Reuber, 1961. J. Natl. Cancer Inst. |
| | 26:891-899. |
| | Pitot HC, et al., 1964. Natl. Cancer Inst. Monogr. 13:229-245. |
| H4-II-E-C3 | ATCC CRL-1600 |
| H4TG | ATCC CRL-1578 |
| H4-II-E | DSM ACC3129 |
| H4-II-Es | DSM ACC3130 |

| | |
|---|---|
| ¹CAP (CEVEC's Amniocyte Production) cells are an immortalized cell line based on primary human amniocytes. They were generated by transfection of these primary cells with a vector containing the functions E1 and pIX of adenovirus 5. CAP cells allow for competitive stable production of recombinant proteins with excellent biologic activity and therapeutic efficacy as a result of authentic human posttranslational modification. | |

Mammalian cells are most preferred, when being established, adapted, and completely cultivated under serum free conditions, and optionally in media, which are free of any protein/peptide of animal origin. Commercially available media such as Ham's F12 (Sigma, Deisenhofen, Germany), RPMI-1640 (Sigma), Dulbecco's Modified Eagle's Medium (DMEM; Sigma), Minimal Essential Medium (MEM; Sigma), Iscove's Modified Dulbecco's Medium (IMDM; Sigma), CD-CHO (Invitrogen, Carlsbad, CA), CHO-S-Invitrogen), serum-free CHO Medium (Sigma), and protein-free CHO Medium (Sigma) are exemplary appropriate nutrient solutions. Any of the media may be supplemented as necessary with a variety of compounds, non-limiting examples of which are recombinant hormones and/or other recombinant growth factors (such as insulin, transferrin, epidermal growth factor, insulin like growth factor), salts (such as sodium chloride, calcium, magnesium, phosphate), buffers (such as HEPES), nucleosides (such as adenosine, thymidine), glutamine, glucose or other equivalent energy sources, antibiotics and trace elements. Any other necessary supplements may also be included at appropriate concentrations that would be known to those skilled in the art. For the growth and selection of genetically modified cells expressing a selectable gene a suitable selection agent is added to the culture medium.

### EXAMPLES

### Methods and Materials

The below examples, unless specified otherwise, were carried out using the following generalized protocol and apparatus for perfusion cell culture. Two different formats of perfusion culture process were employed for the experiments described in the examples. One is executed in a bioreactor model and the other is in a deep-well plate model.

The bioreactor model is set up using a 3-L glass stirred tank bioreactor with a 2 L working volume. The retention device for the bioreactor is a 0.2 µm hollow fiber recirculated by a magnetically levitated centrifugal pump run in the TFF mode. The starting cell density is between 0.5 - 1.0e⁶ cells/mL. The perfusion rate at peak is 2 VVD. Further details of the setup is described by Lin et al. (Biotechnol. Prog., 2017, Vol. 33, No. 4).

The deep-well plate model is carried out using 24 deep-well plates with an 8 day run duration. A starting cell density of 20e⁶ cells/ml is targeted for deep well plate model work. The working volume per well is 3 ml and cells are grown at 33°C with 5% CO₂, 80% humidity, and 200 rpm in an incubator with a 5.0 cm orbit diameter. Medium exchanges are performed daily by centrifuging the plate at 1800 rpm for 5 mins, removing supernatant, and re-suspending the cells in fresh media at 70% volume per day (VVD) exchange rate. Further details of the setup is described by Lin et al. (Biotechnol. Prog., 2017, Vol. 33, No. 4).

### Example 1. The effect of a temperature shift on viability, viable cell density, and specific productivity.

In this example, the bioreactor model is used. Lower temperature shifts are commonly employed in cell culture to limit or inhibit cell growth.

This example therefore investigates the effect of a temperature shift on (A) % percent viability, (B) viable cell density (e⁵ cells/mL), and (C) specific productivity (pg/cell/day). See FIG. 1. Perfusion was started on day 2 of cell culture and gradually was ramped up to an exchange of 2 vessel volumes per day (2VVD). The arrow indicates the day (day 7) when the temperature was shifted (to low) from 37°C to the indicated value in the legend (29°C - squares- or 28°C -triangles-).

In this example, this method has been found to slow growth but found not to affect cell specific productivity. In one cell line (data not shown), the temperature shift negatively impacted product quality, increasing light chain and basic species. As shown in FIG. 1(C), while temperature shifts (to 28°C and 29°C) after day 7 (black arrow) reduce the VCD, the low temperature shifts do not show a positive effect on cell specific productivity (FIG. 1(B)). By contrast, the use of the lipid additives of the invention decrease the VCD while concomitantly increasing the cell specific productivity.

### Example 2. Suppression of cell growth and increase in cell productivity by exogenous linoleic acid.

In this example, the deep-well plate model is used. This example demonstrates that linoleic acid when added to perfusion medium suppresses cell growth and increases cell specific productivity (qₚ) in CHO perfusion cell culture. See FIG. 3.

Linoleic acid at various concentrations 500 µM, 900 µM, 1350 µM and 1800 µM was added to the cell culture media and the effects on (A) viable cell density (e⁵ cells/ml), (B) percent (%) cell viability and (C) specific productivity (qₚ) were determined. Higher concentration of linoleic acid showed the more significant impact on suppressing cell growth and increasing the specific productivity. For example, at day 7, linoleic acid at 500, 900, and 1350 µM demonstrated cell specific productivity which were about the same and which were significantly increased relative to normal perfusion media. Linoleic acid at 1800 µM was similar with regard to viable cell density, viability and specific productivity as arachidonic acid at 500 µM, and it has the most significant difference relative to the normal perfusion media.

This figure also indicates that arachidonic acid at concentration 500 µM can impact cell growth and qₚ in perfusion cell culture.

### Example 3. Suppression of cell growth and increase in cell productivity by exogenous arachidonic acid.

In this example, the deep-well plate model is used. This example analyzes the effect of arachidonic acid when added to perfusion medium. See FIG. 4.

The effect of arachidonic acid on (A) viable cell density (e⁵ cells/ml), (B) percent (%) cell viability and (C) specific productivity (qₚ) of the cell culture was determined.

It can be seen that arachidonic acid concentration at 500 µM added in the normal perfusion media suppressed cell growth up to 31% and increased cell specific productivity by 46% or higher. So, arachidonic acid suppresses cell growth and increases cell specific productivity (qₚ) in perfusion cell culture.

### Example 4. Suppression of cell growth and increase in cell productivity by exogenous arachidonic acid in addition to raised potassium concentration.

In this example, the deep-well plate model is used. This experiment analyzes the effect of arachidonic acid when added to perfusion medium in addition to an already high potassium concentration (i.e., "K-pop" media) See FIG. 5.

The effect of concentrations of arachidonic acid added at 150 µM, 300 µM and 500 µM to a cell culture media having a sodium concentration of approximately 34 mM and a preexisting high potassium concentration of approximately 94 mM, i.e., a low sodium-to-potassium ratio of about 0.4, was determined with respect to(A) viable cell density (e⁵ cells/ml), (B) percent (%) cell viability and (C) specific productivity (qₚ) was determined.

A cell culture medium with arachidonic acid at a concentration from 150 µM to 500 µM in addition to high potassium concentration decreases cell growth and improves cell specific productivity even beyond the effect of high potassium concentration alone.

This experiment demonstrates that arachidonic acid when added to perfusion medium suppresses cell growth and increases cell specific productivity (qₚ) even in addition to a comparable effect by e.g., raised potassium concentration.

A low sodium-to-potassium medium (i.e., or increased potassium concentration) is described, for example, in U.S. provisional application No. 62/479,422.

### Example 5. Suppression of cell growth and increase in cell productivity by exogenous prostaglandin E2 (PGE2).

In this example, the deep-well plate model is used. This experiment analyzes the effect of prostaglandin E2 when added to perfusion medium. See FIG. 6.

FIG. 6 demonstrates the effects of the concentration of arachidonic acid at 500 µM, PGE2 0.001 µM, 10 µM, 20 µM and 60 µM added to the perfusion media on (A) viable cell density (VCD) (e5 cells/ml), (B) percent of cell viability, and (C) specific productivity (pg/cell/day). At each concentration, the VCD and specific productivity (i.e., specific titer), the growth was suppressed cell growth and the specific productivity was increased before day 6 relative to the cell culture in normal perfusion media.

The data also seem to demonstrate that prostaglandin E2 at a concentration greater than 0.001 µM was to a certain extent toxic to cells, as the viability started decreasing on day 6. While some toxicity might be observed above 0.001 µM concentration, this does *per se* not preclude the use of prostaglandin E2 at concentrations above this threshold.

This example demonstrates that prostaglandin E2 when added to perfusion medium suppresses cell growth and increases cell specific productivity (qₚ).

### Example 6. Prostaglandin E2 is regulator of growth suppression and increased cell productivity.

In this example, the deep-well plate model is used. ASA is an inhibitor of COX-1 and COX-2, which are the metabolic enzymes which convert arachidonic acid to synthesize prostaglandin E2 (See FIG. 2). This experiment therefore analyses the effect of acetylsalicylic acid (ASA) when added to perfusion medium. See FIG. 7.

Medium with inhibitor showed higher viable cell density (A) than the normal perfusion medium, with the same effect on specific productivity (C) as normal perfusion medium. Prostaglandin E2 is synthesized from arachidonic acid by cyclooxygenase enzymes 1 and 2 (COX-1 and COX-2), so inhibition of COX-1 and COX-2 with acetylsalicylic acid (ASA) will probably prevent the synthesis of prostaglandin E2 from arachidonic acid.

Without wishing to be bound by this theory, the data suggest that the arachidonic acid - which would otherwise suppress cell growth and increase productivity in the absence of the ASA inhibitor - imposes its effect on the cell culture by being metabolized first to vis-à-vis COX-1 and COX-2 (see FIG. 2) to form prostaglandin E2, which is the active agent. When COX-1 and COX-2 are inhibited by ASA, no prostaglandin E2 is produced, and the arachidonic acid has no effect on suppressing cell growth and increasing productivity.

This experiment demonstrates the effect of acetylsalicylic acid (ASA) on (A) viable cell density, (B) percent viability, and (C) specific productivity in perfusion media with 500 µM of arachidonic acid. The data provide evidence that prostaglandin E2 is effective in triggering cell growth suppression and in increasing cell specific productivity.

This figure also indicates that arachidonic acid at concentration 500 µM can impact cell growth and qₚ in perfusion cell culture.

### Example 3. Suppression of cell growth and increase in cell productivity by exogenous arachidonic acid.

In this example, the deep-well plate model is used. This example analyzes the effect of arachidonic acid when added to perfusion medium. See FIG. 4.

The effect of arachidonic acid on (A) viable cell density (e⁵ cells/ml), (B) percent (%) cell viability and (C) specific productivity (qₚ) of the cell culture was determined.

It can be seen that arachidonic acid concentration at 500 µM added in the normal perfusion media suppressed cell growth up to 31% and increased cell specific productivity by 46% or higher. So, arachidonic acid suppresses cell growth and increases cell specific productivity (qₚ) in perfusion cell culture.

### Example 4. Suppression of cell growth and increase in cell productivity by exogenous arachidonic acid in addition to raised potassium concentration.

In this example, the deep-well plate model is used. This experiment analyzes the effect of arachidonic acid when added to perfusion medium in addition to an already high potassium concentration (i.e., "K-pop" media) See FIG. 5.

The effect of concentrations of arachidonic acid added at 150 µM, 300 µM and 500 µM to a cell culture media having a sodium concentration of approximately 34 mM and a preexisting high potassium concentration of approximately 94 mM, i.e., a low sodium-to-potassium ratio of about 0.4, was determined with respect to(A) viable cell density (e⁵ cells/ml), (B) percent (%) cell viability and (C) specific productivity (qₚ) was determined.

A cell culture medium with arachidonic acid at a concentration from 150 µM to 500 µM in addition to high potassium concentration decreases cell growth and improves cell specific productivity even beyond the effect of high potassium concentration alone.

This experiment demonstrates that arachidonic acid when added to perfusion medium suppresses cell growth and increases cell specific productivity (qₚ) even in addition to a comparable effect by e.g., raised potassium concentration.

A low sodium-to-potassium medium (i.e., or increased potassium concentration) is described, for example, in U.S. provisional application No. 62/479,422, which is incorporated herein by reference.

### Example 5. Suppression of cell growth and increase in cell productivity by exogenous prostaglandin E2 (PGE2).

In this example, the deep-well plate model is used. This experiment analyzes the effect of prostaglandin E2 when added to perfusion medium. See FIG. 6.

FIG. 6 demonstrates the effects of the concentration of arachidonic acid at 500 µM, PGE2 0.001 µM, 10 µM, 20 µM and 60 µM added to the perfusion media on (A) viable cell density (VCD) (e5 cells/ml), (B) percent of cell viability, and (C) specific productivity (pg/cell/day). At each concentration, the VCD and specific productivity (i.e., specific titer), the growth was suppressed cell growth and the specific productivity was increased before day 6 relative to the cell culture in normal perfusion media.

The data also seem to demonstrate that prostaglandin E2 at a concentration greater than 0.001 µM was to a certain extent toxic to cells, as the viability started decreasing on day 6. While some toxicity might be observed above 0.001 µM concentration, this does *per se* not preclude the use of prostaglandin E2 at concentrations above this threshold.

This example demonstrates that prostaglandin E2 when added to perfusion medium suppresses cell growth and increases cell specific productivity (qₚ).

### Example 6. Prostaglandin E2 is regulator of growth suppression and increased cell productivity.

In this example, the deep-well plate model is used. ASA is an inhibitor of COX-1 and COX-2, which are the metabolic enzymes which convert arachidonic acid to synthesize prostaglandin E2 (See FIG. 2). This experiment therefore analyses the effect of acetylsalicylic acid (ASA) when added to perfusion medium. See FIG. 7.

Medium with inhibitor showed higher viable cell density (A) than the normal perfusion medium, with the same effect on specific productivity (C) as normal perfusion medium. Prostaglandin E2 is synthesized from arachidonic acid by cyclooxygenase enzymes 1 and 2 (COX-1 and COX-2), so inhibition of COX-1 and COX-2 with acetylsalicylic acid (ASA) will probably prevent the synthesis of prostaglandin E2 from arachidonic acid.

Without wishing to be bound by this theory, the data suggest that the arachidonic acid - which would otherwise suppress cell growth and increase productivity in the absence of the ASA inhibitor - imposes its effect on the cell culture by being metabolized first to vis-à-vis COX-1 and COX-2 (see FIG. 2) to form prostaglandin E2, which is the active agent. When COX-1 and COX-2 are inhibited by ASA, no prostaglandin E2 is produced, and the arachidonic acid has no effect on suppressing cell growth and increasing productivity.

This experiment demonstrates the effect of acetylsalicylic acid (ASA) on (A) viable cell density, (B) percent viability, and (C) specific productivity in perfusion media with 500 µM of arachidonic acid. The data provide evidence that prostaglandin E2 is effective in triggering cell growth suppression and in increasing cell specific productivity.

## Claims

1. A method of culturing mammalian cells expressing a heterologous protein in a perfusion cell culture, comprising: culturing the mammalian cells in a culture medium comprising an effective amount of one or more lipids or lipid metabolites, wherein the one or more lipids or lipid metabolites comprises 500-2000 µM linoleic acid, 100-600 µM arachidonic acid and/or 0.0001-100 µM prostaglandin E2.

2. The method of claim 1, wherein the culture medium comprises at least two of the lipids or lipid metabolites, preferably (a) wherein the culture medium comprises arachidonic acid at a concentration of 100-300 µM and linoleic acid at a concentration of 500-1800 µM; or (b) wherein the culture medium comprises prostaglandin E2 at a concentration of 0.0001-0.0009 µM in combination with either linoleic acid at a concentration of 500-1800 µM or arachidonic acid at a concentration of 100-150 µM.

3. The method of any one of claims 1 to 2, wherein the culture medium comprises three of the lipids or lipid metabolites.

4. The method of any one of claims 1 to 3, wherein the cell culture on day 2 is changed to a perfusion cell culture, preferably wherein the perfusion rate increases after perfusion has started, preferably wherein the perfusion rate increases from less or equal to 0.5 vessel volumes per day to 5 vessel volumes per day, or from less or equal to 0.5 vessel volumes per day to 2 vessel volumes per day

5. The method of any one of claims 1 to 4, wherein the mammalian cells comprise Chinese Hamster Ovary (CHO) cells, Jurkat cells, 293 cells, HeLa cells, CV-1 cells, or 3T3 cells, or a derivative of any of these cells, wherein said CHO cell can be further selected from the group consisting of a CHO-DG44 cell, a CHO-K1 cell, a CHO DXB11 cell, a CHO-S cell, and a CHO GS deficient cell or a mutant thereof.

6. The method of any one of claims 1 to 5, wherein the heterologous protein is a therapeutic protein, an antibody, or a therapeutically effective fragment thereof, preferably wherein the antibody is a monoclonal antibody or fragment thereof or a bispecific antibody.

7. The method of any one of claims 1 to 6, wherein the lipid or lipid metabolite or combination thereof results in growth suppression and/or increased productivity, preferably
(a) wherein the total production of the heterologous protein produced by the cell culture is increased by at least 5-50% relative the level of total production in a control cell culture that does not include the lipids or lipid metabolites; and/or
(b) wherein the cell specific productivity (pg/cell/day) of the cell culture is increased by at least 5-50% relative the cell specific productivity in a control cell culture that does not include the lipids or lipid metabolites; and/or
(c) wherein cell growth is suppressed at a level which is sufficient to maintain the cells in a steady state having a viable cell density that is at least 5-50% lower relative a control cell culture that does not include the lipids or lipid metabolites.

8. The method of any one of claims 1 to 7, wherein the culture medium is a serum-free perfusion medium, preferably wherein the culture medium is optionally (a) chemically defined, (b) hydrolysate-free, or (c) protein-free but optionally includes insulin and/or insulin-like growth factor.

9. The method of any one of claims 1 to 8, further comprising harvesting the heterologous protein from the cell culture.

10. The method of any one of claims 1 to 9, wherein the one or more lipids or lipid metabolites are added to the cell medium once a cell density of 10 × 10⁶ cells/ml to about 120 × 10⁶ cells/ml is reached.

11. A method of producing a therapeutic protein using the method of any one of the claims 1-10.

12. A method of producing a therapeutic protein from a perfusion cell culture, comprising:
(a) culturing mammalian cells expressing a heterologous protein in a culture medium comprising an effective amount of one or more lipids or lipid metabolites, wherein the one or more lipids or lipid metabolites comprises 500-2000 µM linoleic acid, 100-600 µM arachidonic acid and/or 0.0001-100 µM prostaglandin E2, and
(b) harvesting the heterologous protein from the perfusion cell culture.

13. The method of claim 12, wherein
(a) the effective amount of the one or more lipids or lipid metabolites remains constant during cell culture; and/or
(b) wherein the one or more lipids or lipid metabolites are added to the cell medium once a cell density of 10 × 10⁶ cells/ml to about 120 × 10⁶ cells/ml is reached.

14. A perfusion cell culture medium comprising one or more lipids or lipid metabolites comprising 500-2000 µM linoleic acid, 100-600 µM arachidonic acid and/or 0.0001-100 µM prostaglandin E2, wherein the perfusion cell culture medium is protein-free but optionally includes insulin and/or insulin-like growth factor.

15. Use of the perfusion cell culture medium of claim 14 for culturing mammalian cells in a perfusion culture.

16. Use of the perfusion cell culture medium of claim 14 for suppressing the growth of the perfusion culture and increasing productivity.

## Patentansprüche

1. Ein Verfahren zum Kultivieren von Säugetierzellen exprimierend ein heterologes Protein in einer Perfusionszellkultur, umfassend: Kultivieren der Säugetierzellen in einem Kulturmedium umfassend eine effektive Menge eines oder mehrerer Lipide oder Lipid-Metabolite, wobei das eine oder die mehreren Lipide oder Lipid-Metabolite 500 - 2000 µM Linolsäure, 100 - 600 µM Arachidonsäure und/oder 0,0001 - 100 µM Prostaglandin E2 umfasst.

2. Verfahren nach Anspruch 1, wobei das Kulturmedium mindestens zwei der Lipide oder Lipid-Metabolite umfasst, vorzugsweise (a) wobei das Kulturmedium Arachidonsäure in einer Konzentration von 100 - 300 µM und Linolsäure in einer Konzentration von 500 - 1800 µM umfasst; oder (b) wobei das Kulturmedium Prostaglandin E2 in einer Konzentration von 0,0001 - 0,0009 µM in Kombination mit entweder Linolsäure in einer Konzentration von 500 - 1800 µM oder Arachidonsäure in einer Konzentration von 100 - 150 µM umfasst.

3. Verfahren nach einem der Ansprüche 1 bis 2, wobei das Kulturmedium drei der Lipide oder Lipid-Metabolite umfasst.

4. Verfahren nach einem der Ansprüche 1 bis 3, wobei die Zellkultur an Tag 2 in eine Perfusionszellkultur geändert wird, vorzugsweise worin sich die Perfusionsrate erhöht, nachdem die Perfusion begonnen hat, vorzugsweise worin sich die Perfusionsrate von weniger oder gleich 0,5 Reaktionsgefäßvolumen pro Tag auf 5 Reaktionsgefäßvolumen pro Tag, oder von weniger oder gleich 0,5 Reaktionsgefäßvolumen pro Tag auf 2 Reaktionsgefäßvolumen pro Tag erhöht.

5. Verfahren nach einem der Ansprüche 1 bis 4, wobei die Säugetierzellen Ovarialzellen des chinesischen Hamsters (CHO), Jurkat-Zellen, 293-Zellen, HeLa-Zellen, CV-1-Zellen oder 3T3-Zellen oder ein Derivat von irgendwelchen dieser Zellen umfasst, wobei die CHO-Zellen weiterhin ausgewählt sein können aus der Gruppe bestehend aus einer CHO-DG44-Zelle, einer CHO-K1-Zelle, einer CHO-DXB11-Zelle, einer CHO-S-Zelle, und eine CHO-GS defizienten Zelle oder eine Mutante davon.

6. Verfahren nach einem der Ansprüche 1 bis 5, wobei das heterologe Protein ein therapeutisches Protein, ein Antikörper, oder ein therapeutisch effektives Fragment davon ist, bevorzugt wobei der Antikörper ein monoklonaler Antikörper oder ein Fragment davon oder ein bispezifischer Antikörper ist.

7. Verfahren nach einem der Ansprüche 1 bis 6, wobei das Lipid oder der Lipid-Metabolit oder die Kombination daraus in einer Wachstumshemmung und/oder erhöhten Produktivität resultiert, vorzugsweise
(a) wobei die Gesamtproduktion des in der Zellkultur produzierten heterologen Proteins um mindestens 5 - 50% erhöht ist relativ zu dem Niveau der Gesamtproduktion in einer Kontrollzellkultur, welche die Lipide oder Lipid-Metabolite nicht umfasst; und/oder
(b) wobei die zellspezifische Produktivität (pg/Zelle/Tag) der Zellkultur um mindestens 5 - 50% erhöht ist relativ zu dem Niveau der zellspezifischen Produktivität in einer Kontrollzellkultur, welche die Lipide oder Lipid-Metabolite nicht umfasst; und/oder
(c) wobei das Zellwachstum auf ein Niveau gehemmt wird, das ausreichend ist die Zellen in einem stabilen Zustand mit einer lebenden Zelldichte zu halten, die mindestens 5 - 50% niedriger ist als einer Kontrollzellkultur, welche die Lipide oder Lipid-Metabolite nicht umfasst.

8. Verfahren nach einem der Ansprüche 1 bis 7, wobei das Kulturmedium ein serumfreies Perfusionsmedium ist, vorzugsweise wobei das Kulturmedium optional (a) chemisch definiert, (b) Hydrolysat-frei, oder (c) Protein-frei, aber optional Insulin und/oder einen insulinartigen Wachstumsfaktor umfassend, ist.

9. Verfahren nach einem der Ansprüche 1 bis 8, weiterhin umfassend das Ernten des heterologen Proteins aus der Zellkultur.

10. Verfahren nach einem der Ansprüche 1 bis 9, wobei das eine oder die mehreren Lipide oder Lipid-Metabolite zu dem Zellmedium gegeben werden, sobald eine Zelldichte von 10 × 10⁶ Zellen/ml bis etwa 120 × 10⁶ Zellen/ml erreicht ist.

11. Ein Verfahren zum Herstellen eines therapeutischen Proteins unter Verwendung des Verfahrens nach einem der Ansprüche 1 bis 10.

12. Ein Verfahren zum Herstellen eines therapeutischen Proteins aus einer Perfusionszellkultur, umfassend:
(a) Kultivieren von Säugetierzellen, die ein heterologes Protein exprimieren, in einem Kulturmedium umfassend eine effektive Menge eines oder mehrerer Lipide oder Lipid-Metabolite, wobei das eine oder die mehreren Lipide oder Lipid-Metabolite 500 - 2000 µM Linolsäure, 100 - 600 µM Arachidonsäure und/oder 0,0001-100 µM Prostaglandin E2 umfasst, und
(b) Ernten des heterologen Proteins aus der Perfusionszellkultur.

13. Verfahren nach Anspruch 12, wobei
(a) die effektive Menge des einen oder der mehreren Lipide oder Lipid-Metabolite während der Zellkultur konstant bleibt; und/oder
(b) wobei das eine oder die mehrere Lipide oder Lipid-Metabolite zu dem Zellmedium gegeben werden, sobald eine Zelldichte von 10 × 10⁶ Zellen/ml bis 120 × 10⁶ Zellen/ml erreicht ist.

14. Ein Perfusionszellkulturmedium umfassend ein oder mehrere Lipide oder Lipid-Metabolite umfassend 500 - 2000 µM Linolsäure, 100 - 600 µM Arachidonsäure und/oder 0,0001 - 100 µM Prostaglandin E2, wobei das Perfusionszellkulturmedium Protein-frei ist, aber optional Insulin und/oder einen insulinartigen Wachstumsfaktor umfasst.

15. Verwendung des Perfusionszellkulturmediums nach Anspruch 14 zum Kultivieren von Säugetierzellen in einer Perfusionskultur.

16. Verwendung des Perfusionszellkulturmediums nach Anspruch 14 zum Hemmen des Wachstums der Perfusionskultur und zum Erhöhen der Produktivität.

## Revendications

1. Procédé de culture de cellules mammaliennes exprimant une protéine hétérologue dans une culture cellulaire par perfusion, comprenant : la culture des cellules mammaliennes dans un milieu de culture comprenant une quantité efficace d'un ou plusieurs lipides ou métabolites lipidiques, dans lequel les un ou plusieurs lipides ou métabolites lipidiques comprennent de 500 à 2000 µM d'acide linoléique, de 100 à 600 µM d'acide arachidonique et/ou de 0,0001 à 100 µM de prostaglandine E2.

2. Procédé selon la revendication 1, dans lequel le milieu de culture comprend au moins deux des lipides ou métabolites lipidiques, préférentiellement (a) dans lequel le milieu de culture comprend de l'acide arachidonique à une concentration de 100 à 300 µM et de l'acide linoléique à une concentration de 500 à 1800 µM ; ou (b) dans lequel le milieu de culture comprend de la prostaglandine E2 à une concentration de 0,0001 à 0,0009 µM en combinaison avec de l'acide linoléique à une concentration de 500 à 1800 µM ou de l'acide arachidonique à une concentration de 100 à 150 µM.

3. Procédé selon l'une quelconque des revendications 1 à 2, dans lequel le milieu de culture comprend trois des lipides ou métabolites lipidiques.

4. Procédé selon l'une quelconque des revendications 1 à 3, dans lequel la culture cellulaire le jour 2 est changée en une culture cellulaire par perfusion, préférentiellement dans lequel le taux de perfusion augmente après le début de la perfusion, préférentiellement dans lequel le taux de perfusion augmente de 0,5 volume de cuve par jour ou moins à 5 volumes de cuve par jour, ou de 0,5 volume de cuve par jour ou moins à 2 volumes de cuve par jour.

5. Procédé selon l'une quelconque des revendications 1 à 4, dans lequel les cellules mammaliennes comprennent des cellules d'ovaire de hamster chinois (CHO), des cellules Jurkat, des cellules 293, des cellules HeLa, des cellules CV-1 ou des cellules 3T3, ou un dérivé de l'une quelconque de ces cellules, dans lequel ladite cellule CHO peut être en outre choisie parmi le groupe constitué d'une cellule CHO-DG44, d'une cellule CHO-K1, d'une cellule CHO DXB11, d'une cellule CHO-S et d'une cellule CHO déficiente en GS ou d'une forme mutante de celles-ci.

6. Procédé selon l'une quelconque des revendications 1 à 5, dans lequel la protéine hétérologue est une protéine thérapeutique, un anticorps ou un fragment thérapeutiquement efficace de celui-ci, préférentiellement dans lequel l'anticorps est un anticorps monoclonal ou un fragment de celui-ci ou un anticorps bispécifique.

7. Procédé selon l'une quelconque des revendications 1 à 6, dans lequel le lipide ou le métabolite lipidique ou une combinaison de ceux-ci entraîne une suppression de la croissance et/ou une productivité accrue, préférentiellement
(a) dans lequel la production totale de la protéine hétérologue produite par la culture cellulaire est augmentée d'au moins 5 à 50 % par rapport au niveau de production totale dans une culture cellulaire témoin qui ne comprend pas les lipides ou les métabolites lipidiques ; et/ou
(b) dans lequel la productivité spécifique des cellules (pg/cellule/jour) de la culture cellulaire est augmentée d'au moins 5 à 50 % par rapport à la productivité spécifique des cellules dans une culture cellulaire témoin qui ne comprend pas les lipides ou les métabolites lipidiques ; et/ou
(c) dans lequel la suppression de la croissance cellulaire se produit à un niveau qui est suffisant pour maintenir les cellules dans un état d'équilibre ayant une densité cellulaire viable qui est inférieure d'au moins 5 à 50 % par rapport à une culture cellulaire témoin qui ne comprend pas les lipides ou les métabolites lipidiques.

8. Procédé selon l'une quelconque des revendications 1 à 7, dans lequel le milieu de culture est un milieu de perfusion sans sérum, préférentiellement dans lequel le milieu de culture est éventuellement (a) défini chimiquement, (b) sans hydrolysat ou (c) sans protéine, mais comprenant éventuellement de l'insuline et/ou un facteur de croissance analogue à l'insuline.

9. Procédé selon l'une quelconque des revendications 1 à 8, comprenant en outre la récolte de la protéine hétérologue à partir de la culture cellulaire.

10. Procédé selon l'une quelconque des revendications 1 à 9, dans lequel les un ou plusieurs lipides ou métabolites lipidiques sont ajoutés au milieu cellulaire une fois qu'une densité cellulaire de 10 × 10⁶ cellules/ml à environ 120 × 10⁶ cellules/ml est atteinte.

11. Procédé de production d'une protéine thérapeutique à l'aide du procédé selon l'une quelconque des revendications 1 à 10.

12. Procédé de production d'une protéine thérapeutique à partir d'une culture cellulaire par perfusion, comprenant :
(a) la culture de cellules mammaliennes exprimant une protéine hétérologue dans un milieu de culture comprenant une quantité efficace d'un ou plusieurs lipides ou métabolites lipidiques, dans lequel les un ou plusieurs lipides ou métabolites lipidiques comprennent de 500 à 2000 µM d'acide linoléique, de 100 à 600 µM d'acide arachidonique et/ou de 0,0001 à 100 µM de prostaglandine E2, et
(b) la récolte de la protéine hétérologue à partir de la culture cellulaire par perfusion.

13. Procédé selon la revendication 12, dans lequel
(a) la quantité efficace des un ou plusieurs lipides ou métabolites lipidiques reste constante pendant la culture cellulaire ; et/ou
(b) dans lequel les un ou plusieurs lipides ou métabolites lipidiques sont ajoutés au milieu cellulaire une fois qu'une densité cellulaire de 10 × 10⁶ cellules/ml à environ 120 × 10⁶ cellules/ml est atteinte.

14. Milieu de culture cellulaire par perfusion comprenant un ou plusieurs lipides ou métabolites lipidiques comprenant de 500 à 2000 µM d'acide linoléique, de 100 à 600 µM d'acide arachidonique et/ou de 0,0001 à 100 µM de prostaglandine E2, dans lequel le milieu de culture cellulaire par perfusion est sans protéine, mais comprend éventuellement de l'insuline et/ou un facteur de croissance analogue à l'insuline.

15. Utilisation du milieu de culture cellulaire par perfusion selon la revendication 14 pour la culture de cellules mammaliennes dans une culture par perfusion.

16. Utilisation du milieu de culture cellulaire par perfusion selon la revendication 14 pour la suppression de la croissance de la culture par perfusion et l'augmentation de la productivité.
